# EUROPEAN PATENT APPLICATION

(11) **EP 2 090 890 A1**
(43) Date of publication of application: **19.08.2009**
(21) Application number: 08151519.9
(22) Date of filing: 15.02.2008
(51) Int. Cl.: G01N 33/574

(54) **RBM3 as a marker for breast cancer prognosis**

(71) Applicant: Atlas Antibodies AB, 106 91 Stockholm (SE)
(72) Inventor: Uhlén, Mathias, 182 79 Stocksund (SE); Pontén, Fredrik, 752 37 Uppsala (SE); Jirström, Karin, 216 18 Limhamn (SE)
(74) Representative: Henriksson, Dan Ragnar Mikael

(57) **Abstract**

The present invention provides means, such as a method, for determining whether a prognosis for a mammalian subject having a breast cancer is better than a reference prognosis. The method comprises the steps of: providing a sample earlier obtained from the subject; evaluating the amount of RBM3 protein present in at least part of said sample, and determining a sample value corresponding to said evaluated amount; comparing the sample value obtained in step b) with a reference value associated with said reference prognosis; and, if said sample value is higher than said reference value, concluding that the prognosis for said subject is better than said reference prognosis.

## Description

### Field of the invention

The present invention relates to the field of breast cancer prognostics and diagnostics, and in particular to molecular markers having prognostic or diagnostic value and uses thereof.

### Background of the invention

### Cancer

Cancer is one of the most common causes of disease and death in the western world. In general, incidence rates increase with age for most forms of cancer. As human populations continue to live longer, due to an increase of the general health status, cancer will affect an increasing number of individuals. The cause of most common cancer types is still at large unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and antisocial behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. It is important to emphasize that a malignant tumor does not only consist of the transformed tumor cells themselves but also surrounding normal cells which act as a supportive stroma. This recruited cancer stroma consists of connective tissue, blood vessels and various other normal cells, e.g. inflammatory cells, which act in concert to supply the transformed tumor cells with signals necessary for continued tumor growth.

The most common forms of cancer arise in somatic cells and are predominantly of epithelial origin, e.g. prostate, breast, colon, urothelial and skin, followed by cancers originating from the hematopoetic lineage, e.g. leukemia and lymphoma, neuroectoderm, e.g. malignant gliomas, and soft tissue tumors, e.g. sarcomas.

### Cancer diagnostics and prognostics

Microscopic evaluation of a tissue section taken from a tumor remains the golden standard for determining a diagnosis of cancer. For microscopic diagnosis, biopsy material from suspected tumors is collected and examined under the microscope. To obtain a firm diagnosis, the tumor tissue is fixated in formalin, histo-processed and paraffin embedded. From the resulting paraffin block, tissue sections can be produced and stained using both histochemical, i.e. hematoxylin-eosin staining, and immunohistochemical methods. The surgical specimen is then evaluated with pathology techniques, including gross and microscopic analysis. This analysis forms the basis for assigning a specific diagnosis, i.e. classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e. how far the tumor has invaded and imposed growth into surrounding normal tissues, whereas the N stage and M stage describe how the tumor has developed into metastasis, with the N stage describing spread of tumor to lymph nodes and the M stage describing growth of tumor in other distant organs. Early stages include: T0-1, N0, M0, representing localized tumors with negative lymph nodes. More advanced stages include: T1-4, N0-4, M0, localized tumors with more widespread growth and T1-4, N1-4, M0, tumors that have metastasized to lymph nodes and T1-4, N1-4, M1, tumors with a metastasis detected in a distant organ. Staging of tumors is often based on several forms of examinations, including surgical, radiological and histopathological analyses. In addition to the staging, there is also a classification system to grade the level of malignancy for most tumor types. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and Elston-Ellis grading for breast carcinomas.

Accurate staging and grading is crucial for a correct diagnosis and provides an instrument to predict a prognosis. The diagnostic and prognostic information for a specific tumor subsequently determines an adequate therapeutic strategy for a given cancer patient. The most commonly used method, in addition to histochemical staining of tissue sections, to obtain more information regarding a tumor is immunohistochemical staining. IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be important to support the accurate diagnosis, including staging and grading, of a primary tumor as well as in the diagnostics of metastases of unknown origin. The most commonly used antibodies in clinical practice today include antibodies against cell type "specific" proteins, e.g. PSA (prostate), MelanA (melanocytes), Thyroglobulin (thyroid gland) and antibodies recognizing intermediate filaments (epithelial, mesenchymal, glial) cluster of differentiation (CD) antigens (hematopoetic, sub-classification of lympoid cells) and markers of malignant potential, e.g. Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites all add important information. However, most of these analyses still represent basic research and have yet to be evaluated and standardized for the use in clinical medicine.

In order for physicians to give a cancer patient the right type of treatment as early as possible, the provision of new molecular markers that allows for more accurate stratification of cancer patients into different risk categories is crucial. We are still far a way from this type of individually tailored treatment. In summary, there is a great demand for new means to advance prognostics and staging of cancer.

### Breast cancer

Breast cancer is the second most common form of cancer worldwide and by far the most frequent cancer of women. Data from the GLOBOCAM 2002 database presented by Parkin *et al* reveal 1.15 million new cases in 2002 and 0.41 million deaths during the same period (Parkin DM et al (2005) CA Cancer J Clin 55, 74-108). If detected at an early stage, the prognosis is relatively good for a patient living in a developed country, with a general five-year survival rate of 73%, compared to 57% in a developing country. The incidence is slowly increasing and about one in every nine women in the developed world will get breast cancer in her lifetime. Although lifestyle changes related to female steroid hormones, including exposure to exogenous hormones, affect the risk of developing breast cancer, these factors only make up for a small fraction of the etiology, and the gain of preventive manipulation would probably be low. The decreased mortality is due to earlier detection by mammography screening and the use of modern adjuvant systemic treatment.

### Treatment

Since its introduction in the late seventies, breast-conserving therapy, combining breast conserving surgery and postoperative radiotherapy, has become the primary treatment of choice in women where radical removal of the tumor can be combined with a good cosmetic result. Mastectomy is still preferable in some patients, i.e. women with small breasts, large tumors (> 4 cm) or multifocal/multicentric disease.

Axillary dissection is primarily performed for diagnostic purposes and removal of at least 10 lymph nodes gives a good staging guidance with 97-98% sensitivity (Axelsson CK eta/(1992) Eur J Cancer 28A:1415-8; Recht A and Houlihan MJ (1995) Cancer 6(9):1491-1512) However, the next step towards minimal surgery in the treatment of primary cancer has been the introduction of the sentinel node biopsy technique with mapping of axillary lymph nodes instead of axillary lymph node clearance, which is associated with a high complication rate. This technique was introduced as a consequence of the knowledge that most of the lymphatic drainage to the axilla from the breast initially passes through one (or a few) lymph node(s)- the sentinel node(s)- supporting that analysis of this lymph node may be a sufficient indicator of axillary node status (Veronesi U et al (2003) New Engl J Med 349(6): 546-53.)

The concept of breast cancer as a systemic disease, i.e. the presence of disseminating micro-metastases at the time of diagnosis that may explain treatment failure after locoregional therapy, paved the way for adjuvant randomized trials in the 1970s, including endocrine therapy and chemotherapy. Adjuvant polychemotherapy is standard treatment for hormone-receptor negative patients with high risk of recurrence, irrespective of nodal status. A beneficial effect on both overall- and relapse-free survival has been demonstrated, especially in premenopausal patients (EBCTCG (1998) Lancet 352(9132; 930-42). For patients with hormone-responsive disease, i.e. estrogen receptor (ER) and/or progesterone receptor (PR) positive disease, adjuvant polychemotherapy has been delivered in combination with endocrine therapy as sequential chemo-endocrine therapy. Adjuvant chemotherapy also induces amenorrhea, causing a secondary endocrine effect in addition to the cytotoxic (Pagani O et al (1998) Eur J Cancer 34(5):632-40).

Endocrine therapy is recommended for patients with hormone receptor positive tumors irrespective of age, stage and menopausal status.

In hormone-responsive premenopausal patients, ovarian ablation by surgery or irradiation, or ovarian suppression by LHRH agonists are efficient adjuvant treatment modalities (Emens LA and Davidson NA (2003) Clin Ca Res (1 Pt 2): 468S-94S). In postmenopausal patients, ovarian ablation has no place, since the primary source of estrogen is not from ovarian synthesis but from the conversion of androstenedione to estrone and estradiol in peripheral tissues including the breast.

Tamoxifen is a selective estrogen receptor modulator (SERM) with an agonistic effect on the ER, making it a suitable treatment for advanced breast cancer in both pre- and postmenopausal women. Five years of tamoxifen as adjuvant treatment after primary surgery clearly reduces the breast cancer mortality in patients with ER positive (ER+) tumors, irrespective of lymph node status (EBCTCG (1998) Lancet 351(9114):1451-67). While tamoxifen has a protective effect against cardiovascular disease, the risk of developing endometrial cancer is increased, due to an agonistic effect on the ER in the endometrium (EBCTCG (2005) Lancet 365(9472):1687-717)

Aromatase inhibitors (Als) function by inhibiting aromatase, the enzyme converting androgens into estrogens. Als are not suitable for treatment of premenopausal women, as it stimulates the ovaries to an increased androgen production through the hypothalamus and pituitary gland. Als can be given as adjuvant treatment to postmenopausal women, either alone or following tamoxifen treatment and they have been shown to significantly reduce the mortality, possibly even more if given alone (Howell A et al (1995) Lancet 345(8941):29-30, Ellis MJ and Rigden CE (2006) Curr Med Res Opin 22(12):2479-87, Coates AS et al (2007) J Clin Oncol 25(5):486-92). However, this therapy is relatively new and the long-term side effects are not yet fully known (Buzdar A et al (2006) Lancet Oncol 7(8):633-43), but the most important are cardiovascular complications and osteoporosis.

Newly developed pure anti-estrogens such as fulvestrant, which completely blocks the ER, are currently only used in advanced breast cancer and not in the adjuvant setting (Rutqvist LE (2004) Best Pract Res Clin Endocrinol Metab 18(1): 81-95).

Adjuvant endocrine therapy has no place in hormone receptor negative breast cancer, although some studies indicate that some ER negative (ER-), i.e. ERa negative (ERa-), tumors respond to tamoxifen treatment (EBCTCG (1998) Lancet 351:1451-1467)

The HER2/neu gene is over expressed in about 20% of all and in up to 70% of low differentiated breast cancers (Berger MS et al (1988) Cancer Res 48(5): 1238-43, Borg A et al (1990) Cancer Res 50(14): 4332-7). Patients with HER2 overexpressing tumors may benefit from treatment with the monoclonal antibody trastuzumab. Experimental data support a relationship between HER2 overexpression and resistance to endocrine treatment (Shou J et al (2004) J Natl Cancer Inst 96(12):926-35) while clinical data are not consistent (Borg A et al (1994) Cancer Lett 81(2):137-44, De Placido S et al (2003) Clin Ca Res 9(3):1039-46, Rydén L et al (2005) J Clin Oncol 23(21):4695-704).

### Breast cancer diagnostics

Morphologic criteria are still essential for the establishment of a breast cancer diagnosis, both *in situ* and invasive cancer, and although such criteria are more or less subjective, they are not easily substituted. Among invasive breast carcinomas, *invasive ductal carcinoma* is the most common tumor type (∼80 %) and *lobular carcinoma* is the second largest entity (∼10-15%). *Tubular* and *medullary* carcinomas are other distinct types with lower prevalence (WHO, Histological typing of breast tumors, in International histological classification of tumors no:2, 1981, WHO, Geneva).

### Prognostic and treatment predictive factors

A correct histological classification of the tumor type may be of prognostic relevance, since certain subtypes, such as medullary carcinomas, in general have a more favorable prognosis. Nevertheless, assessment of the histological grade using the Nottingham Histological Grade (NHG) system is still an important prognostic tool (Elston CW and Ellis IO (1991) 19(5):403-10, Sundquist M et al (1999) Breast Cancer Res Treat 53(1):1-8).

The majority of breast cancers are hormone receptor responsive, i.e. express the ER and/or PR. The action of estrogen is mediated by the two receptors, ERa and ERβ. ERα together with PR are routinely assessed in order to select patients for endocrine therapy, and tumors with >10% nuclear positivity are considered positive. ERα is today considered the most important predictor of tamoxifen response. However, there are studies that indicate that PR positivity may even be a more powerful predictor of tamoxifen response than ERα. A study of premenopausal patients randomly treated with tamoxifen revealed that a high, >75% nuclear fraction, PR level was significantly correlated with an increased recurrence free and overall survival, irrespectively of ERα status. At a lower PR level, <75%, no positive effect was observed (Stendahl M et al (2006) Clin Cancer Res 12:4614-18). Yu et al recently studied the predictive value of PR for adjuvant endocrine therapy, and found that older patients (≥ 60 years) with ERα+/PR+ tumors had a significantly longer disease free survival when treated with tamoxifen than patients that received no adjuvant treatment. In younger patients (<60 years), no significant effect was observed (Yu KD et al (2007) The Breast 16:307-315). Interestingly, a report from the ATAC trial (postmenopausal women treated with arimidex, tamoxifen or in combination), showed that the recurrence rate was halved for anastrozole-treated patients with ERα+/PR-tumors over the follow-up period of 6 years, compared to patients treated with tamoxifen (Dowsett M et al (2005) J Clin Oncol 23(30:7512-7).

The role of ERβ in breast cancer is not yet fully clarified, although recent studies implicate that ERβ-expression may be associated with a better tamoxifen response (Borgquist S et al, (2008) J Clin Pathol 61(2):197-203), particularly in ERα- tumors (Gruvberger-Saal SK et al (2007) Clin Cancer Res. 13:1987-1994). However, determination of ERβ is today generally not considered clinically relevant.

A major problem to day is that 30-40% of the ERα positive (ERα+) patients do not respond to tamoxifen treatment (Riggins RB et al (2007) Cancer Letters 1:1-24, Gruvberger-Saal SK et al (2007) Clin Cancer Res. 13:1987-1994), which results in unnecessary treatment. In addition, a fraction of the ERα- patients do respond to tamoxifen treatment, and the reason for that is currently not known. Gruvberger-Saal suggests that ERβ-expression may be a positive predictor of tamoxifen response in ERα- patients (Gruvberger-Saal SK et al (2007) Clin Cancer Res. 13:1987-1994.

HER2 status is also assessed routinely, primarily by IHC and in cases with moderate expression, gene amplification status is determined by fluorescence in situ hybridization (FISH) analysis. Patients with a HER2 positive tumor may be treated with Trastuzumab (Herceptin).

Breast cancer is a truly heterogeneous disease and despite the increasing understanding of its nature, the arsenal of available prognostic and treatment predictive markers is still not sufficient and some patients will therefore receive unnecessary treatment while others will not get sufficient or even ineffective treatment. Additional molecular markers are needed in order to better define different subgroups of breast cancer and increase the options for tailored therapies.

### Endpoint analysis

Endpoint analysis for trials with adjuvant treatments for cancer gives important information on how the patients respond to a certain therapy. Overall survival (OS) has long been considered the standard primary endpoint. OS takes in to account time to death, irrespective of cause, e.g. if the death is due to cancer or not. Loss to follow-up is censored and regional recurrence, distant metastases, second primary breast cancers, and second other primary cancers are ignored.

To day, an increasing number of effective treatments available in many types of cancer have resulted in the need for surrogate endpoints to allow for a better evaluation of the effect of adjuvant treatments. Thus, the much longer follow-up required to demonstrate that adjuvant treatments improve OS is often complemented with other clinical endpoints that gives an earlier indication on how successful the treatment is.

In the present disclosure, patient cohorts are evaluated by OS analysis, however two surrogate endpoints are also considered, namely recurrence-free survival (RFS) and breast cancer-specific survival (BCSS). Analysis of RFS includes time to any event related to the same cancer, i.e. all cancer recurrences and deaths from the same cancer are events. Also distant, local and regional metastases as well as breast cancer specific death are considered. On the other hand, second primary same cancers and other primary cancers are ignored, as well as contralateral breast cancer. Deaths from other cancers, non-cancer-related deaths, treatment-related deaths, and loss to follow-up are censored observations. Breast cancer-specific survival (BCSS) includes time to death caused by the same cancer, whether due to the original tumor or to a second primary same cancer. This endpoint analysis is part of the RFS analysis, but may be of interest to analyze separately.

### RBM3

The gene encoding RNA binding motif protein 3 (RBM3) was initially isolated from fetal brain tissue (Derry JM et al (1995) Hum Mol Genet 4:2307-2311). RBM3 is upregulated early in response to cold-shock (Danno S et al (1997) Biochem Biophys Res Commun 236:804-807) and may protect against certain cell death pathways (Kita H el al (2002) Hum Mol Genet. 11:2279-87). The RBM3 gene is together with two other RNA binding proteins, RBMX and RBM10, all located on the X-chromosome. A recent study revealed a significant association between expression of the X-chromosome related RBM-genes and the proapoptotic Bax gene in breast cancer tissue (Martinez-Arribas F et al (2006) J Cell Biochem 97:1275-82). Another protein identified with RBM domains is RBM5/LUCA15, which is reported to play a role during cell proliferation and apoptosis and has been found down regulated in both lung and breast cancer (Mourtada-Maarabouni M and Williams GT (2006) Exp Cell Res. 312:1745-52). However, there are no studies on the tumor-specific expression of RBM3 protein in breast cancer, particularly in relation to disease outcome.

### Disclosure of the invention

It is an object of the present invention to provide new tools for establishing a prognosis for a breast cancer patient.

For this and other objects apparent to the skilled person from the present disclosure, the present invention provides, in its different aspects, new means for determining a prognosis for a mammalian subject having a breast cancer for determining whether such a subject should undergo a breast cancer treatment and for treatment of such a subject. The present invention is defined by the appending claims. Thus, according to a first aspect, there is provided a method for determining whether a prognosis for a mammalian subject having a breast cancer is better than a reference prognosis, comprising the steps of:
a) providing a sample earlier obtained from the subject;
b) evaluating the amount of RBM3 protein present in at least part of the sample, and determining a sample value corresponding to the evaluated amount;
c) comparing the sample value obtained in step b) with a reference value associated with the reference prognosis; and, if the sample value is higher than the reference value,
d) concluding that the prognosis for the subject is better than the reference prognosis.

Regarding step b), an increase in the amount of RBM3 protein typically results in an increase in the sample value, and not the other way around.

In an embodiment, the above method may comprise the additional step:
e) if the sample value is lower than, or equal to, the reference value, concluding that the prognosis for the subject is worse than the reference prognosis.

This first aspect of the present invention is based on the previously unrecognized fact that the amount of RBM3 protein present in samples earlier obtained from a subject having a breast cancer may serve as a disease status indicator in the subject. More particularly, the present invention identifies for the first time, in subjects suffering from a breast cancer, a correlation between an amount of RBM3 protein on the one hand and a prognosis for survival on the other. Typically, high RBM3 protein values have been shown to correlate with a good prognosis in these subjects, probably due to a less aggressive or low-risk form of the cancer. The present invention based on RBM3 protein expression as a cancer status indicator has a number of benefits. In general, early identification of the aggressiveness a breast cancer is of vital importance as it helps a physician selecting an appropriate treatment strategy. For example, by identifying less aggressive forms at an early stage, over-treatment may be avoided. As a further example, the RBM3 protein, as a marker for which a certain level of expression is correlated with a certain pattern of disease progression, has a great potential for example in a panel for differential diagnostics of a primary tumor.

In the context of the present disclosure, a reference value being "associated" with a reference prognosis refers to the reference value being assigned a corresponding reference prognosis, based on empirical data and/or clinically relevant assumptions.

In the present disclosure, different RBM3 protein values (sample values) corresponding to various prognoses are presented. Typically, a high sample value is associated with a better prognosis than a low sample value. In the above method, the sample value is compared to a reference value, and if the sample value is higher than the reference value, it is concluded that the prognosis for the subject is better than a reference prognosis associated with the reference value.

Consequently, the methods of the above aspects may be adapted to a given reference value. In such case, starting from a given sample value which under certain circumstances is considered to be relevant, a reference value which is equal to, or lower than, that sample value, may be selected. Subsequently, a reference prognosis being associated with that reference value may be established. Guided by the present disclosure, the person skilled in the art understands how to establish a reference prognosis which corresponds to a given reference value. For example, the relation between sample values and survival data in a group of breast cancer patients may be examined as in Examples, section 4, 5 or 6, below, and the procedure described therein may be adapted to a given reference value. Then, a prognosis corresponding to the given reference value may be selected as the reference prognosis.

Also, the methods of the above aspects may be adapted to a given reference prognosis. In such case, starting from a given reference prognosis which under certain circumstances is considered to be relevant, for example for selecting an appropriate treatment strategy, a corresponding reference value may be established. Guided by the present disclosure, the person skilled in the art understands how to establish a reference value which corresponds to a given reference prognosis. For example, the relation between sample values and survival data in a group of cancer patients may be examined as in Examples, section 4, 5 or 6, below, but the procedure described therein is adapted to establish reference values corresponding to a given reference prognosis. For example, different reference values may be tested until one which correlates with the given reference prognosis is found.

In embodiments of the methods of the above aspect, the reference prognosis may be based on a previous prognosis obtained by an examination of the same subject or population of subjects. Alternatively, or as a complement, the reference prognosis may be based on a background risk in the general population, a statistical prognosis/risk or an assumption based on an examination of the subject. Such examination may comprise the subject's age, general condition, sex, race and/or medical status and history, such as cancer history or breast cancer status. For example, a reference prognosis may be established by a physician taking into regard the subject's cancer history, the stage of the tumor, the morphology of the tumor, the location of the tumor, the presence and spread of metastases and/or further cancer characteristics.

Further, the reference prognosis may be specifically adapted to the hormone receptor status, such as the ER status and/or the PR status, of the subject, i.e. one reference prognosis may apply to hormone receptor positive, e.g. ER+ and/or PR+, subjects only and another reference prognosis may apply to hormone receptor negative, e.g. ER- or PR-, subjects only. The person skilled in the art understands how to determine a hormone receptor status of the subject. Also, examples of hormone receptor status determinations are given below in connection with the endocrine treatment product aspects.

Also, the reference prognosis may for example be a reference probability of survival, such as five-year survival, ten-year survival or 15-year survival. As further examples, the survival may be an over-all survival, a recurrence free survival or a breast cancer specific survival.Accordingly, the prognosis for said subject of the methods of the above aspect may also be a probability of survival, such as five-year survival, ten-year survival or 15-year survival, wherein the survival may for example be an over-all survival, a recurrence free survival or a breast cancer specific survival. Consequently, the prognosis being better than the reference prognosis may correspond to a probability of survival which is higher than a reference probability of survival.

As shown in the attached figures, the correlation between high RBM3 protein levels and a good prognosis is particularly accentuated if recurrence free survival (RFS) is analyzed. Consequently, in embodiments of the methods of the above aspects, the prognosis may be a probability of recurrence free survival. If the prognosis is a probability of recurrence free survival it follows that the reference prognosis preferably should be a probability of recurrence free survival.

As shown in the attached figures, the prognosis for subjects having high RBM3 protein levels are generally better than those for subjects having low RBM3 protein levels. Provided the teachings of the present disclosure, a physician may consider the prognosis of an RBM3 protein high subject being so favorable that no adjuvant breast cancer therapy is necessary. The present disclosure may thus relieve subjects from over treatment.

Consequently, as an alternative embodiment of the first aspect, there is provided a method for determining whether a mammalian subject having a breast cancer is not in need of an adjuvant breast cancer treatment:
a) providing a sample from the subject;
b) evaluating the amount of RBM3 protein present in at least part of the sample, and determining a sample value corresponding to the evaluated amount;
c) comparing the sample value obtained in step b) with a reference value; and, if the sample value is higher than the reference value,
d) concluding that the subject is not in need of the adjuvant breast cancer treatment.

Regarding step b), an increase in the amount of RBM3 protein typically results in an increase in the sample value, and not the other way around.

In the context of the present disclosure, "not in need of an adjuvant breast cancer treatment" refers to the case wherein the benefits of the adjuvant breast cancer treatment do not compensate for the disadvantages of the same. The benefits of the adjuvant breast cancer treatment refers to a higher probability of survival or recovery if undergoing the treatment than if not undergoing the treatment. The "disadvantages of the adjuvant breast cancer treatment" refers to the drawbacks, such as side-effects, pain or other inconveniences and costs.

For example, the reference value of step c) may be associated with a reference prognosis, and consequently, a prognosis interval for the subject, i.e. the information that the prognosis for the subject is better than the reference prognosis, may be obtained. The connection between reference values and reference prognoses is described above.

By obtaining RBM3 protein information, such as a prognosis, regarding a subject according to the above embodiment, a physician may conclude that the subject is not in need of an adjuvant breast cancer treatment. As an example, the reference prognosis or reference value may be adapted to suit an individual case to make such judgment. Various conditions to which the reference value and/or prognosis may be adapted are described above.

As a related, alternative embodiment of the first aspect, there is provided a non-treatment strategy method for a subject having a breast cancer, comprising:
a) providing a sample from the subject;
b) evaluating the amount of RBM3 protein present in at least part of the sample, and determining a sample value corresponding to the evaluated amount;
c) comparing the sample value obtained in step b) with a reference value; and, if the sample value is higher than the reference value,
d) refraining from treating the subject with an adjuvant breast cancer treatment.

Regarding step b), an increase in the amount of RBM3 protein typically results in an increase in the sample value, and not the other way around.

For example, the reference value of step c) may be associated with a reference prognosis, and consequently, a prognosis interval for the subject, i.e. the information that the prognosis for the subject is better than the reference prognosis, may be obtained. The connection between reference values and reference prognoses is described above.

By obtaining RBM3 protein information, such as a prognosis, regarding a subject according to the above embodiment, a physician may refrain from treating the subject with an adjuvant breast cancer treatment. As an example, the reference prognosis or reference value may be adapted to suit an individual case to make such judgment. Various conditions to which the reference value and/or prognosis may be adapted are described above.

For example, the refraining of step d) may be a refraining of at least one week from the completion of steps a) - c), such as at least one month from the completion of steps a) - c), such as at least three months from the completion of steps a) - c), such as at least six months from the completion of steps a) - c), such as at least one year from the completion of steps a) - c), such as at least two years from the completion of steps a) - c).

Alternatively, the refraining of step d) may be a refraining until the next time the method is performed or until recurrence of a breast cancer tumor.

In embodiments of the two related methods above, the sample may be an earlier obtained sample.

In conclusion, the two related methods above are based on the inventors insight that a high RBM3 protein values may indicate that an adjuvant breast cancer treatment is unnecessary.

As an example, giving an RBM3 protein high subject adjuvant chemotherapy may be unnecessary. Consequently, in embodiments of the two related methods above, the adjuvant breast cancer treatment may be chemotherapy. For example, the chemotherapy may be mono- or polychemotherapy, such as treatment with CMF (cyclophosphamide, methotrexate, 5-fluorouracil), FEC (fluorouracil, epirubicin, cyclofosfamid) and/or taxanes.

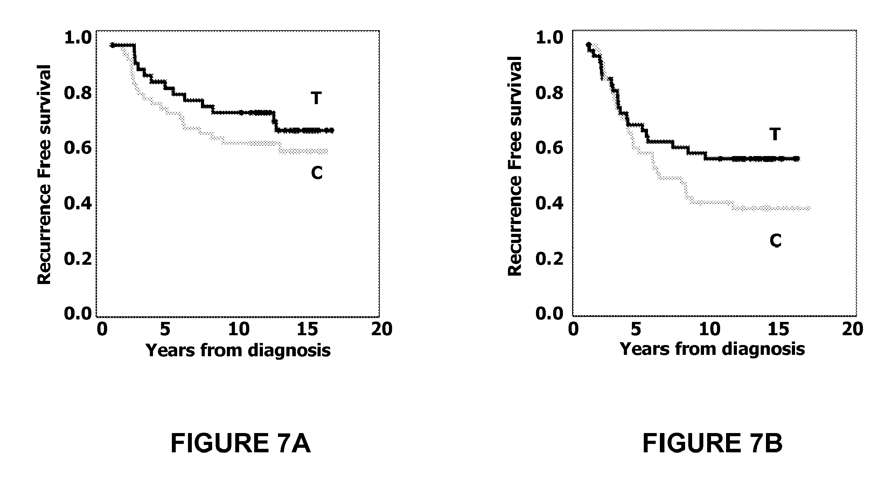

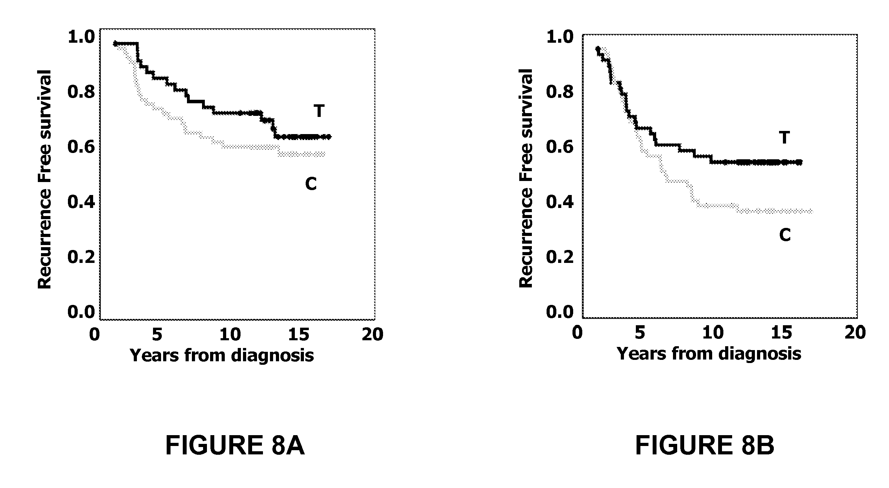

As described in Examples, section 6 and shown in figures 7 and 8, the positive effect of tamoxifen treatment is less apparent in RBM3 protein high subjects than in RBM3 protein low subjects. Consequently, in embodiments of the two related methods above, the adjuvant breast cancer treatment may be an endocrine treatment, such as tamoxifen treatment or an aromatase inhibitor treatment. In the context of the present disclosure, an "endocrine treatment" refers to a systemic treatment with an anti-estrogenic effect. Further, "anti-estrogenic effect" refers to suppression of estrogen production or inhibition of estrogen effects in the body. In the art, an endocrine treatment is sometimes referred to as an anti-hormonal treatment.

Further, in embodiments of the two related methods above, the adjuvant breast cancer treatment may a combination of a chemotherapy, such as one or more of the above-mentioned chemotherapies, and an endocrine therapy. An example of such a combination is sequential chemo-endocrine therapy, wherein the subjects is first treated with a chemotherapy which is followed by an endocrine treatment. As an example, such sequential chemo-endocrine therapy has traditionally been given to hormone receptor positive (HR+) subjects. By utilizing one of the two related methods above, this HR+ group may be relieved from the chemotherapy part and/or the endocrine therapy part of such a sequential chemo-endocrine therapy.

As an alternative aspect of the present disclosure, there is provided a method of treatment of a mammalian subject having a breast cancer, comprising:
a) providing a sample from the subject;
b) evaluating the amount of RBM3 protein present in at least part of the sample, and determining a sample value corresponding to the evaluated amount;
c) comparing the sample value obtained in step b) with a reference value; and, if the sample value is equal to or lower than the reference value,
d) treating the subject with an adjuvant breast cancer treatment.

Regarding step b), an increase in the amount of RBM3 protein typically results in an increase in the sample value, and not the other way around.

It follows from the discussion above that the prognosis for RBM3 protein low patients are worse than those for RBM3 protein high patients, and consequently, that the need for adjuvant treatment is generally higher for RBM3 protein low patients.

In the context of the present disclosure, the "reference value" refers to a predetermined value found to be relevant for making decisions, or drawing conclusions, regarding the prognosis, or a suitable treatment strategy, for the subject.

In embodiments of the above method, the adjuvant breast cancer treatment may be a chemotherapy, an endocrine treatment or a combination thereof, such as a sequential chemo-endocrine therapy.

As an example, the the chemotherapy may be mono- or polychemotherapy, such as treatment with CMF (cyclophosphamide, methotrexate, 5-fluorouracil), FEC (fluorouracil, epirubicin, cyclofosfamid) and/or taxanes.

As described in Examples, section 6 and shown in figures 7 and 8, the positive effect of tamoxifen treatment is more apparent in RBM3 protein low subjects than in RBM3 protein high subjects. Consequently, in embodiments of the above method, the adjuvant breast cancer treatment may be an endocrine treatment, such as tamoxifen treatment or an aromatase inhibitor treatment.

Further, in embodiments of the above method, the adjuvant breast cancer treatment may be a combination of a chemotherapy, such as one or more of the above-mentioned chemotherapies, and an endocrine therapy. An example of such a combination is sequential chemo-endocrine therapy, wherein the subjects is first treated with a chemotherapy which is followed by an endocrine treatment. As an example, such sequential chemo-endocrine therapy has traditionally been given to HR+ subjects. By utilizing the method above, this HR+ group may be relieved from the chemotherapy part and/or the endocrine therapy part of such a sequential chemo-endocrine therapy.

When deciding on a suitable treatment strategy for a breast cancer subject, the physician responsible for the treatment may take several parameters into account, such as the result of an immunohistochemical evaluation, patient age, menopausal status, hormone receptor status, general condition and medical history, such as breast cancer history. To be guided in the decision, the physician may perform RBM3 protein test, or order an RBM3 test performed, according to an embodiment of a method aspects presented above.

As another alternative embodiment of the first aspect, there is provided a method for establishing a prognosis for a mammalian subject having a breast cancer:
a) providing a sample from the subject;
b) evaluating the amount of RBM3 protein present in at least part of the sample, and determining a sample value corresponding to the evaluated amount;
c) correlating the sample value of step b) to the prognosis for the subject.

Regarding step b), an increase in the amount of RBM3 protein typically results in an increase in the sample value, and not the other way around.

In an embodiment of the above method, the sample may be an earlier obtained sample.

The correlating of step c) refers to any way of associating survival data to the obtained sample value so as to establish a prognosis for the subject.

In the context of the present disclosure, "a mammalian subject having a breast cancer" refers to a mammalian subject having a primary or secondary breast cancer tumor or a mammalian subject which have had a tumor removed from a breast, wherein the removal of the breast tumor refers to killing or removing the breast tumor by any appropriate type of surgery or therapy.

Step b) of the methods of the above aspects involve evaluating the amount of RBM3 protein present in at least part of the sample, and determining a sample value corresponding to the amount. The "at least part of the sample" refers to a relevant part, or relevant parts, of the sample. The person skilled in the art understands which part or parts that are relevant under the circumstances present when performing the method. For example, if evaluating at a sample comprising cells, the skilled person may only consider the tumor cells, or only the nuclei of tumor cells, of the sample.

Further, in step b) an amount is evaluated and a sample value corresponding to the amount is determined. Consequently, an exact measurement of the amount of RBM3 protein is not required for obtaining the sample value. For example, the amount of RBM3 protein may be evaluated by visual inspection of a stained sample and the sample value may then be categorized as a high or low based on the evaluated amount.

In embodiments of the methods of the above aspects, the sample may be a body fluid sample, such as blood, plasma, serum, cerebral fluid, urine, or exudate. Alternatively, the sample may be a cytology sample or a stool sample.

In further embodiments of the methods of the above aspects, the sample may be a tissue sample. As an example, the tissue sample may be derived from a primary breast tumor or secondary tumor (metastasis) of the subject. Further, the tissue sample may be a tumor sample from a breast of the subject.

In embodiments of the methods of the above aspects, the evaluation of step b) may be limited to evaluating the amount of RBM3 protein expression in tumor cells of the tissue sample. The inventors have found that RBM3 is expressed in the nucleus. Consequently, in embodiments of the methods of the above aspects, the evaluation of step b) may be limited to evaluating the amount of RBM3 protein expression in the nucleus of tumor cells of the tissue sample.

In embodiments of the methods of the above aspects, the subject may be a human and/or a female.

Further, in embodiments of the methods of the above aspects, the subject may be a premenopausal or postmenopausal female.

As shown in the figures, the correlation between high RBM3 protein levels and a good prognosis is particularly accentuated in subjects having hormone receptor positive (HR+) cancers, such as estrogen receptor positive (ER+) cancers or progesterone receptor positive cancers (PR+). Consequently, in embodiments of the methods of the above aspects, the breast cancer may be HR+, such as ER+ or PR+, ER+, PR+, or ER+ and PR+.

T1N0M0 subjects, especially premenopausal T1N0M0 subjects, is a frequently over-treated group of breast cancer subjects. The findings of the present disclosure may relieve this group from unnecessary treatment. Accordingly, in embodiments of the methods of the above aspects, the breast cancer may be a breast cancer previously classified as T1 N0M0 according to the TNM staging system.

In Examples, section 6, below, the prognostic value of RBM3 is shown using samples derived from patients having stage II (pT2 N0 M0, pT1 N1 M0 or pT2 N1 M0) primary breast carcinoma. Consequently, in embodiments of the methods of the above aspects, the breast cancer may be a breast cancer previously classified as pT2 N0 M0, pT1 N1 M0 or pT2 N1 M0 according to the TNM staging system.

Also, in embodiments of the methods of the above aspects, the breast cancer may be a node negative cancer. A "node negative cancer" refers to a cancer that has not spread to the lymph nodes. The above methods, especially those relating to the non-treatment of the subject, may be particularly useful for such less advanced stages of breast cancer.

A sample value of RBM3 protein being higher than the reference value, or a subject from which such sample value is obtained, is sometimes referred to herein as "RBM3 protein high". Further, a sample value of RBM3 protein being lower than, or equal to, the reference value, or a subject from which such sample value is obtained, is sometimes referred to herein as "RBM3 protein low".

A reference value found to be relevant for the provision of a prognosis for a subject having a breast cancer, or for making treatment decisions regarding such subjects, for use as comparison with the sample value from the subject, may be provided in various ways. With the knowledge of the teachings of the present disclosure, the skilled artisan can, without undue burden, provide relevant reference values for performing the methods of the above aspects.

The person performing the methods of the above aspects may, for example, adapt the reference value to desired prognostic information. For example, the reference value may be adapted to yield the most significant prognostic information, e.g. the largest separation between the RBM3 "high" survival curve and the RBM3 "low" survival curve (see the figures). Examples of reference values that may yield a large separation is a nuclear fraction of 25 %, which is the reference value of cohort I presented below, or a nuclear fraction of 75 %, which is the reference value of cohort II and III below.

Alternatively, the reference value may be adapted to identify a group of subjects having a predetermined prognosis, e.g. the group of subjects having a likelihood of a five year recurrence free survival of at least 80%.

In embodiments of the methods of the above aspects, the reference value may correspond to the amount of RBM3 protein expression in a healthy tissue, such as healthy breast tissue or stroma tissue, of the subject of the method. As another example, the reference value may be provided by the amount of RBM3 protein expression measured in a standard sample of normal tissue from another, comparable subject. As another example, the reference value may be provided by the amount of RBM3 protein expression measured in a reference sample comprising tumor cells, such as a reference sample of tumor tissue. As another example, the reference value may be provided by the amount of RBM3 protein measured in a reference sample comprising cells expressing a predetermined amount of RBM3 protein.

As another example, the reference value may be provided by the amount of RBM3 protein expression measured in a reference sample comprising cell lines, such as cancer cell lines, expressing a predetermined, or controlled, amount of RBM3 protein. The person skilled in the art understands how to provide such cell lines, for example guided by the disclosure of Rhodes et al. (2006) The biomedical scientist, p 515-520.

Accordingly, in embodiments of the methods of the above aspects, the reference value may be a predetermined value corresponding to the amount of RBM3 protein in a reference sample.

In the context of the present disclosure, the terms "sample value" and "reference value" are to be interpreted broadly. The quantification of RBM3 protein to obtain these values may be done via automatic means, via a scoring system based on visual or microscopic inspection of samples, or via combinations thereof. However, it is also possible for a skilled person, such as a person skilled in the art of histopathology, to determine the sample and reference values merely by inspection of e.g. tissue slides that have been stained for RBM3 protein expression. The determination of the sample value being higher than the reference value may thus correspond to the determination, upon visual or microscopic inspection, that a sample tissue slide is more densely stained and/or exhibit a larger fraction of stained cells than is the case for a reference tissue slide. In this case, the sample and reference values are thought of as mental values that the skilled person determines upon inspection and comparison.

The skilled person may categorize a sample as being high or low in RBM3 protein expression, wherein the sample is categorized as high in RBM3 protein expression if it contains more RBM3 protein than a previously inspected reference sample. Such evaluation may be assisted by staining the sample, and, if necessary, the reference sample, with a staining solution comprising e.g. antibodies specific for RBM3 protein.

One alternative for the quantification of RBM3 protein expression in a sample is the determination of the fraction of cells in the sample that exhibit RBM3 protein expression over a certain level. The fraction may for example be: a "cellular fraction", wherein the RBM3 protein expression of the whole cells is taken into account; a "cytoplasmic fraction", wherein the RBM3 protein expression of only the cytoplasms of the cells is taken into account, or the "nuclear fraction", wherein the RBM3 protein expression of only the nuclei of the cells is taken into account. This determination may for example be performed as described below in the Examples, section 3, with reference to "nuclear fraction". The "nuclear fraction" corresponds to the percentage of relevant cells in a sample that exhibits a positive staining in the nucleus, wherein a medium or distinct and strong immunoreactivity in the nucleus is considered positive and no or faint immunoreactivity in the nucleus is considered negative. The person skilled in the art of pathology understands which cells that are relevant under the conditions present when performing the method and may determine a nuclear fraction based on his general knowledge and the teachings of the present disclosure. The relevant cells may for example be tumor cells. Further, the skilled artisan understands how to perform corresponding measurements employing the "cellular fraction" or the "cytoplasmic fraction". In embodiments of the methods of the above aspects, the criterion for the conclusion in step d) is a sample value for the nuclear fraction of RBM3 positive cells, i.e. a "nuclear fraction", which is higher than the reference value of 1 %, such as higher than 5 %, such as higher than 10 %, such as higher than 15 %, such as higher than 20 %, such as higher than 25 %, such as higher than 30 %, such as higher than 35 %, such as higher than 40 %, such as higher than 50 %, such as higher than 55 %, such as higher than 60 %, such as higher than 65 %, such as higher than 70 %, such as higher than 75 %, such as higher than 80 %, such as higher than 85 %, such as higher than 90 %, such as higher than 95 %.

In alternative or complementing embodiments of the methods of the above aspects, the reference value of step c) is a nuclear fraction of 1 % or higher, such as 5 % or higher, such as 10 % or higher, such as 15 % or higher, such as 20 % or higher, such as 25 % or higher, such as 30 % or higher, such as 35 % or higher, such as 40 % or higher, such as 45 % or higher, such as 50 % or higher, such as 55 % or higher, such as 60 % or higher, such as 65 % or higher, such as 70 % or higher, such as 85 % or higher, such as 90 % or higher, such as 95 % or higher.

In cohorts I and II-III presented below, nuclear fractions of 25 % and 75 %, respectively, were found to give significant results regarding prognoses. Consequently, in embodiments of the methods of the above aspects, the reference value of step c) is a nuclear fraction of 5 - 95 %, such as 10 - 90 %, such as 15 - 85 %, such as 20 - 80 %, such as 20-30 %, 25 - 75 % or 70 - 80%.

Often, e.g. in cohorts I-III presented below, nuclear fractions a categorized as < 2 %, 2-25 %, > 25 - 75 % or > 75 %. Consequently, especially if such categorization is used, the reference value may be selected from a nuclear fraction of 25 % or 75 %.

Another alternative for the quantification of RBM3 expression in a sample is the determination of the over-all staining intensity of the sample. The intensity may for example be: a "cellular intensity", wherein the RBM3 protein expression of the whole cells is taken into account; a "cytoplasmic intensity", wherein the RBM3 protein expression of only the cytoplasms of the cells is taken into account, or the "nuclear intensity", wherein the RBM3 protein expression of only the nuclei of the cells is taken into account. Nuclear intensity is subjectively evaluated in accordance to standards used in clinical histo-pathological diagnostics. Outcome of a nuclear intensity determination is classified as: absent = no over-all immunoreactivity in relevant cells of the sample, weak = faint over-all immunoreactivity in relevant cells of the sample, moderate = medium over-all immunoreactivity in relevant cells of the sample, or strong = distinct and strong over-all immunoreactivity in relevant cells of the sample. The person skilled in the art understands which cells that are relevant under the conditions present when performing the method and may determine a nuclear intensity based on his general knowledge and the teachings of the present disclosure. The relevant cells may for example be tumor cells. This determination may for example be performed as described below in the Examples, section 3, definition of "nuclear intensity". Further, the skilled artisan understands how to perform corresponding measurements employing the "cellular intensity" or the "cytoplasmic intensity". In embodiments of the methods of the above aspects, the criterion for the conclusion in step d) may be a sample value for staining intensity of a sample, i.e. a nuclear intensity, which is higher than absent nuclear intensity, such as higher than weak intensity, such higher than moderate intensity. In alternative or complementing embodiments of the methods of the above aspects, the reference value of step c) may be an absent nuclear intensity of RBM3 protein expression or higher, such as a weak nuclear intensity of RBM3 protein expression or higher, such as moderate nuclear intensity of RBM3 protein expression.

Further, in embodiments of the methods of the above aspects, the reference value may be constituted of two values, wherein the criterion for the conclusion in step d) is a sample value being higher than any of these two values.

Alternatively, in embodiments of the methods of the above aspects, the reference value may be a coincidence of a fraction value and an intensity value, such as a nuclear fraction value and a nuclear intensity value.

Also, in embodiments of the methods of the above aspects, the reference value may be a function of a nuclear fraction value and a nuclear intensity value. For example, such function may be a staining score. The "staining score" is calculated as described in Examples, section 3 and table 1 below. For example, the reference value may be a staining score of 0 or higher, such as 1 or higher, such as 2.

The person skilled in the art realizes that other reference values being an intensity value or a fraction value also fall within the scope of the present invention. Likewise, the person skilled in the art realizes that other combinations of fractions and intensities also fall within the scope of the present invention. Consequently, the reference value may involve two, and possibly even more, criteria.

Guided by the present disclosure, and especially Examples, sections 3-6 below, a person skilled in the art, e.g. a pathologist, understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of RBM3 protein for establishing the appearance of a certain fraction or intensity.

However, a reference sample may not only be used for the provision of the actual reference value, but also for the provision of an example of a sample with an amount of RBM3 protein that is higher than the amount corresponding to the reference value. As an example, in histochemical staining, such as in immunohistochemical staining, the skilled artisan may use a reference sample for establishing the appearance of a stained sample with a high amount of RBM3 protein, e.g. a positive reference. Subsequently, the skilled artisan may assess the appearances of samples with lower amounts of RBM3, such as the appearance of a sample with an amount of RBM3 corresponding to the reference value. In other words, the skilled artisan may use a reference sample to create a mental image of a reference value corresponding to an amount of RBM3 protein which is lower than that of the reference sample. Alternatively, or as a complement, in such assessments, the skilled artisan may use another reference sample having a low amount of RBM3, or essentially lacking RBM3, for establishing the appearance of such sample, e.g. as a "negative reference".

Consequently, in the evaluation, the skilled artisan may use a reference sample for establishing the appearance of a sample with a high amount of RBM3 protein. Such reference sample may be a sample comprising tissue expressing a high amount of RBM3 protein, such as a sample comprising tumor tissue from breast having a pre-established high expression of RBM3 protein.

Further, the reference sample may provide an example of a strong nuclear intensity (NI). With the knowledge of the appearance of a sample with strong nuclear intensity, the skilled artisan may then divide samples into the NI categories presented in Examples, section 4, below, i.e. absent, weak, moderate and strong. This division may be further assisted by a reference sample essentially lacking RBM3 protein (negative reference), i.e. a reference sample providing an absent nuclear intensity. Also, the reference sample may provide an example of a sample with a nuclear fraction (NF) of 75 % or higher. With the knowledge of the appearance of a sample with more than 75 % positive cells, the skilled artisan may then evaluate the nuclear fraction of other samples having e.g. a lower percentage of positive cells. This division may be further assisted by a reference sample essentially lacking RBM3 protein (negative reference), i.e. a reference sample providing a low NF (e.g. < 5%, such as ≤ 2%), or a NF of 0.

As mentioned above, cell lines expressing a controlled amount of RBM3 protein may be used as the reference, in particular as a positive reference.

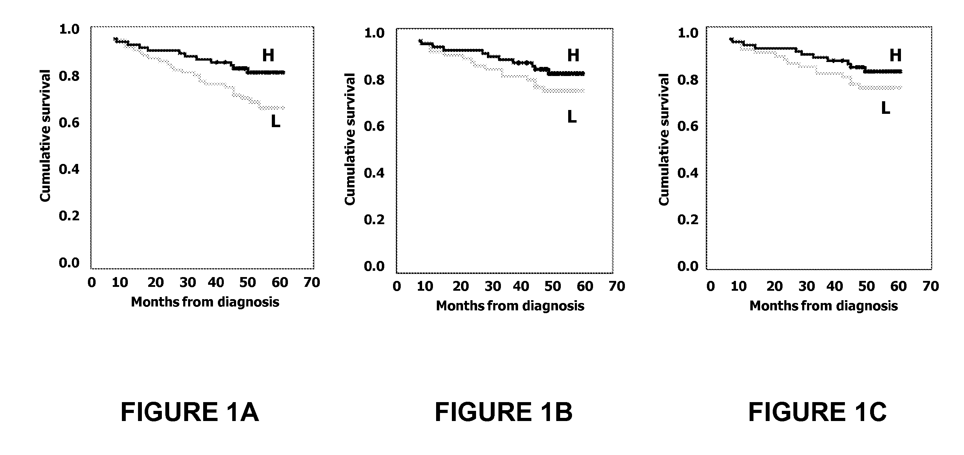

As discussed above, the methods according to the above aspects may be adapted to a selected reference value, such as one of the reference values presented above, and the reference prognosis will in such case be a consequence of the selected reference value. As a non-limiting example, if the reference value of Cohort I (Examples, Section 49) is used, i.e. NF = 25 %, an associated reference prognoses may be derived from figure 1 by looking at the "low" curve, i.e. the curve representing the patients having sample values which are equal to, or lower than, the reference value NF = 25 %. At a given time from diagnosis, the corresponding cumulative survival according to the "low" curve may be read from the figure, e.g. 66 % after 5 years (60 months) for the group of all patients (fig 1A), which results in a reference prognosis being a probability of five-year survival of 66 %. Consequently, patients of the same group having sample values which are higher than the reference value NF = 25 % have a probability of five year survival which is higher than 66 %. Using the same logic, the associated reference prognosis may be a probability of five-year survival of 75 % for the group of ER+ patients (fig 1 B), and a probability of five-year survival of 76 % for the group of HR+ patients (fig 1 C). Further associated reference prognoses may be derived from the other figures. The skilled artisan understands how to identify reference prognoses associated with reference values, and may do so without undue burden.

The skilled person will recognize that the usefulness of the present invention is not limited to the quantification of any particular variant of the RBM3 protein present in the subject in question, as long as the protein is encoded by the relevant gene and presents the relevant pattern of expression. As a non-limiting example, the RBM3 protein has an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the RBM3 protein has an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

In embodiments of the methods of the aspects above, the RBM3 protein may be detected and/or quantified through the application to a sample of a detectable and/or quantifiable affinity ligand, which is capable of specific or selective interaction with the RBM3 protein. The application of the affinity ligand is performed under conditions that enable binding of the affinity ligand to any RBM3 protein in the sample.

To concretize, in embodiments of the methods of the aspects above, steb b) may comprise:
b1) applying to the sample a quantifiable affinity ligand capable of selective interaction with the RBM3 protein to be quantified, the application being performed under conditions that enable binding of the affinity ligand to any RBM3 protein present in the sample;
b2) removing non-bound affinity ligand; and
b3) quantifying any affinity ligand remaining in association with the sample to evaluate the amount.

In the context of the present disclosure, a "selective interaction" refers to a

It is regarded as within the capabilities of those of ordinary skill in the art to select or manufacture the proper affinity ligand and to select the proper format and conditions for detection and/or quantification, once the connection between RBM3 protein and breast cancer is known through the teaching of the present disclosure. Nevertheless, examples of affinity ligands that may prove useful, as well as examples of formats and conditions for detection and/or quantification, are given below for the sake of illustration.

Thus, in some embodiments of the methods of the above aspects, an affinity ligand is used, which is selected from the group consisting of antibodies, fragments thereof and derivatives thereof, i.e. affinity ligands based on an immunoglobulin scaffold. Antibodies comprise monoclonal and polyclonal antibodies of any origin, including murine, human and other antibodies, as well as chimeric antibodies comprising sequences from different species, such as partly humanized mouse antibodies. Polyclonal antibodies are produced by immunization of animals with the antigen of choice, whereas monoclonal antibodies of defined specificity can be produced using the hybridoma technology developed by Köhler and Milstein (Köhler G and Milstein C (1976) Eur. J. Immunol. 6:511-519). Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al (1991) Biochemistry 30:6803-6805); camelid heavy-chain dimers (Hamers-Casterman C et al (1993) Nature 363:446-448) and single variable domains (Cai X and Garen A (1996) Proc. Natl. Acad. Sci. U.S.A. 93:6280-6285; Masat L et al (1994) Proc. Natl. Acad. Sci. U.S.A. 91:893-896), and single domain scaffolds like e.g. the New Antigen Receptor (NAR) from the nurse shark (Dooley H et al (2003) Mol. Immunol. 40:25-33) and minibodies based on a variable heavy domain (Skerra A and Plückthun A (1988) Science 240:1038-1041).

Polyclonal and monoclonal antibodies, as well as their fragments and derivatives, represent the traditional choice of affinity ligands in applications requiring selective biomolecular recognition, such as in the detection and/or quantification of RBM3 protein according to the method aspects above. However, those of skill in the art know that, due to the increasing demand of high throughput generation of specific binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against RBM3 for use according to the present disclosure, are staphylococcal protein A and domains thereof and derivatives of these domains, such as protein Z (Nord K et al (1997) Nat. Biotechnol. 15:772-777); lipocalins (Beste G et al (1999) Proc. Natl. Acad. Sci. U.S.A. 96:1898-1903); ankyrin repeat domains (Binz HK et al (2003) J. Mol. Biol. 332:489-503); cellulose binding domains (CBD) (Smith GP et al (1998) J. Mol. Biol. 277:317-332; Lehtiö J et al (2000) Proteins 41:316-322); γ crystallines (Fiedler U and Rudolph R, WO01/04144); green fluorescent protein (GFP) (Peelle B et al (2001) Chem. Biol. 8:521-534); human cytotoxic T lymphocyte-associated antigen 4 (CTLA-4) (Hufton SE et al (2000) FEBS Lett. 475:225-231; Irving RA et al (2001) J. Immunol. Meth. 248:31-45); protease inhibitors, such as Knottin proteins (Wentzel A et al (2001) J. Bacteriol. 183:7273-7284; Baggio R et al (2002) J. Mol. Recognit. 15:126-134) and Kunitz domains (Roberts BL et al (1992) Gene 121:9-15; Dennis MS and Lazarus RA (1994) J. Biol. Chem. 269:22137-22144); PDZ domains (Schneider S et al (1999) Nat. Biotechnol. 17:170-175); peptide aptamers, such as thioredoxin (Lu Z et al (1995) Biotechnology 13:366-372; Klevenz B et al (2002) Cell. Mol. Life Sci. 59:1993-1998); staphylococcal nuclease (Norman TC et al (1999) Science 285:591-595); tendamistats (McConell SJ and Hoess RH (1995) J. Mol. Biol. 250:460-479; Li R et al (2003) Protein Eng. 16:65-72); trinectins based on the fibronectin type III domain (Koide A et al (1998) J. Mol. Biol. 284:1141-1151; Xu L et al (2002) Chem. Biol. 9:933-942); and zinc fingers (Bianchi E et al (1995) J. Mol. Biol. 247:154-160; Klug A (1999) J. Mol. Biol. 293:215-218; Segal DJ et al (2003) Biochemistry 42:2137-2148).

The above mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al (2008) Protein Eng Des Sel 1-9, Harvey BR et al (2004) PNAS 101 (25):913-9198), microbead display (Nord O et al (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the methods of the above aspects, an affinity ligand may be used, which is a non-immunoglobulin affinity ligand derived from any of the protein scaffolds listed above, or an oligonucleotide molecule.

In some embodiments of the methods of the above aspects, an affinity ligand capable of selective interaction with the RBM3 protein is detectable and/or quantifiable. The detection and/or quantification of such an affinity ligand may be accomplished in any way known to the skilled person for detection and/or quantification of binding reagents in assays based on biological interactions. Thus, any affinity ligand, as described above, may be used quantitatively or qualitatively to detect the presence of the RBM3 protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with RBM3 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). Further, the affinity ligands may also be labeled with fluorescent semiconductor nanocrystals (Quantum dots). Quantum dots have superior quantum yield and are more photostable compared to organic fluorophores and are therefore more easily detected (Chan et al (2002) Curr Opi Biotech. 13: 40-46). The different types of labels can be conjugated to an affinity ligand using various chemistries, e.g. the amine reaction or the thiol reaction. However, other reactive groups than amines and thiols can be used, e.g. aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its RBM3 protein target may involve visualizing techniques, such as light microscopy or immunofluoresence microscopy. Other methods may involve the detection via flow cytometry or luminometry.

A biological sample, such as a tissue sample (biopsy), for example from breast tissue, may be removed from the subject for detection and/or quantification of RBM3 protein. Alternatively, the biological sample, such as the biopsy, may be an earlier obtained sample. If using an earlier obtained sample, no steps of any one of the embodiments of the methods of the above aspects are practiced on the human or animal body. The affinity ligand may be applied to the biological sample for detection and/or quantification of the RBM3 marker protein. This procedure enables not only detection of RBM3 protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from chemical reaction. Those of skill in the art are aware that a variety of different protocols can be modified in order for proper detection and/or quantification.

In embodiments of the methods of the above aspects, a biological sample may be immobilized onto a solid phase support or carrier, such as nitrocellulose or any other solid support matrix capable of immobilizing any RBM3 protein present in the biological sample applied to it. Some well-known solid state support materials useful in the present invention include glass, carbohydrate (e.g. Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand specific to RBM3 may be applied, e.g. as described in Examples, sections 2 and/or 3, of the present disclosure. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art will be able to determine operative and optimal assay conditions for each determination by routine experimentation.

Consequently, in embodiments of the methods of the above aspects, the quantifiable affinity ligand of b1) may be detected using a secondary affinity ligand capable of recognizing the quantifiable affinity ligand. The quantification of b3) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the RBM3 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the RBM3 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the current invention are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize RBM3 protein by detection of radioactivity *in vivo* or *in vitro.* Radionuclear scanning with e.g. gamma camera, magnetic resonance spectroscopy or emission tomography function for detection *in vivo* and *in vitro,* while gamma/beta counters, scintillation counters and radiographies are also used *in vitro.*

As a further aspect of the present disclosure, there is provided a kit for carrying out a method according to any embodiment of the above aspects, which comprises
a) a quantifiable affinity ligand capable of selective interaction with an RBM3 protein; and
b) reagents necessary for quantifying the amount of the affinity ligand.

Various components of the kit according to the kit aspect may be selected and specified as described above in connection with the method aspects of the present disclosure.

Thus, the kit according to the invention comprises an affinity ligand against RBM3, as well as other means that help to quantify the specific and/or selective affinity ligand after it has bound specifically and/or selectively to RBM3. For example, the kit may contain a secondary affinity ligand for detecting and/or quantifying a complex formed by any RBM3 protein and the affinity ligand capable of selective interaction with an RBM3 protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g. endogenous enzyme block solution to decrease the background staining and/or counterstaining solution to increase staining contrast, that are commonly used in immunoassay reagent kits.

Thus, in embodiments of the kit aspect, the quantifiable affinity ligand is selected from the group consisting of antibodies, fragments thereof and derivatives thereof. As an example, such quantifiable affinity ligand may be obtainable by a process comprising a step of immunizing an animal, such as a rabbit, with a protein whose amino acid sequence comprises the sequence SEQ ID NO:1 or a sequence which is at least 85 % identical to SEQ ID NO:1, preferably the sequence SEQ ID NO:1. Such immunization may for example be performed as described in Examples, section 2, below.

Alternatively, the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers. As a further alternative, the quantifiable affinity ligand is an oligonucleotide molecule.

Further, in embodiments of the kit aspect, the detectable affinity ligand may comprise a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit according to the kit aspect may also advantageously comprise a reference sample for provision of, or yielding, the reference value to be used for comparison with the sample value. Preferably, the reference sample comprises a predetermined amount of RBM3 protein. Such a reference sample may for example be constituted by a tissue sample having a predetermined amount of RBM3 protein, which may then be used by the person of skill in the art to determine the RBM3 expression status in the sample being studied, by manual, such as ocular, or automated comparison of expression levels in the reference sample and the subject sample. As another example, the reference sample may comprise cell lines, such as cancer cell lines, expressing a predetermined, or controlled, amount of RBM3 protein. The person skilled in the art understands how to provide such cell lines, for example guided by the disclosure of Rhodes et al. (2006) The biomedical scientist, p 515-520. As an example, the cell lines may be formalin fixed. Also, such formalin fixed cell lines may be paraffin embedded.

The wording "reference sample for provision of the reference value" is to be interpreted broadly in the context of the kit aspect. Such reference sample may comprise an amount of RBM3 protein actually corresponding to the reference value, but it may also comprise an amount of RBM3 protein corresponding to a value being higher than the reference value. In the latter case, the "high" value may be used by a person performing the method as an upper reference (positive reference) for assessing, e.g. the appearance of, a reference value which is lower than the "high" value. The skilled person understands how to do such an assessment. Further, as an alternative or a complementing example, the skilled person may use another reference sample comprising a low amount of RBM3 protein for provision of a "low" value in such an assessment, e.g. as a negative reference.

Consequently, in embodiments of the kit aspect, the reference sample may comprise an amount of RBM3 protein corresponding to a reference value. As an example, the reference sample may comprise an amount of RBM3 protein corresponding to a nuclear fraction of 1 % or higher, such as 5 % or higher, such as 10 % or higher, such as 15 % or higher, such as 20 % or higher, such as 25 % or higher, such as 30 % or higher, such as 35 % or higher, such as 40 % or higher, such as 45 % or higher, such as 50 % or higher, such as 55 % or higher, such as 60 % or higher, such as 65 % or higher, such as 70 % or higher, such as 75 % or higher, such as 80 % or higher, such as 85 % or higher, such as 90 % or higher, such as 95 % or higher. Also, the reference sample may comprise an amount of RBM3 protein corresponding to a nuclear fraction of 5 - 95 %, such as 10 - 90 %, such as 15 - 85 %, such as 20 - 80 %, such as 20-30 %, 25 - 75 % or 70 - 80 %. Alternatively, or as a complement, the reference sample may comprise an amount of RBM3 protein corresponding to an absent nuclear intensity of RBM3 protein expression or higher, such as a weak nuclear intensity of RBM3 protein expression or higher, such as moderate nuclear intensity of RBM3 protein expression.

Consequently, the reference sample may comprise an amount of RBM3 protein corresponding to a nuclear fraction of 25 % or higher and a weak nuclear intensity of RBM3 expression or higher. Further, the reference sample may comprise an amount of RBM3 protein corresponding a staining score of 0, 1 or 2. The provision of nuclear fraction values or nuclear intensity values is discussed above in connection with the method aspects.

Further, in alternative embodiments of the kit aspect, the reference sample may comprise an amount of RBM3 protein corresponding to a value being higher than the reference value. In these embodiments, the reference sample may for example comprise an amount of RBM3 protein corresponding to a nuclear fraction of 75 % or higher and/or a strong nuclear intensity of RBM3 expression.

In other alternative embodiments of the kit aspect, the reference sample may comprise an amount of RBM3 protein corresponding to a value being lower than the reference value.

Also, in alternative or complementing embodiments of the kit aspect, the kit may comprise: a reference sample comprising an amount of RBM3 protein corresponding to a predetermined reference value; a reference sample comprising an amount of RBM3 protein corresponding to a value being higher than a predetermined reference value; and/or a reference sample comprising an amount of RBM3 protein corresponding to a value being lower than a predetermined reference value.

Consequently, embodiments of the kit may comprise: a first reference sample comprising an amount of RBM3 protein being higher than a predetermined reference value; and a second reference sample comprising an amount of RBM3 protein being lower than the predetermined reference value.

In embodiments of the kit aspect, the reference sample may be a tissue sample, such as a tissue sample adapted to ocular or microscopic evaluation. As an example, the tissue reference sample may be fixated in paraffin or buffered formalin and/or histo-processed to µm-thin sections that are mounted on microscopic glass-slides. The tissue reference sample may be further adapted to staining with affinity ligands, such as antibodies, for an RBM3 protein.

Consequently, in embodiments of the kit aspect, the reference sample may be adapted to directly, or indirectly, provide any relevant reference value, such as any one of the reference values discussed above.

Accordingly, further embodiments of the reference sample of the kit aspect are discussed above in connection with the reference values and reference samples of the method aspects.

RBM3 protein may be used in the production of tools that may be used for measuring and/or evaluating clinically relevant amounts of RBM3 protein. Accordingly, as a further aspect of the present disclosure, there is provided an RBM3 protein, or an antigenically active fragment thereof, for manufacture of a prognostic agent for establishing a prognosis for a mammalian subject having a breast cancer. In the context of the present disclosure, an "antigenically active fragment" of an RBM3 protein is a fragment of sufficient size to be useful for the generation of an affinity ligand, e.g. an antibody, which will interact with an RBM3 protein comprising the fragment. For example, the manufacture may be accomplished as described in Examples, Section 2.

The amino acid sequence of the RBM3 protein may for example comprise a sequence selected from: i) SEQ ID NO:1; and ii) a sequence which is at least 85 % identical to SEQ ID NO:1. In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1. Also, the RBM3 protein may for example comprise a sequence selected from: i) SEQ ID NO:2; and ii) a sequence which is at least 85 % identical to SEQ ID NO:2. In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

As a related aspect thereof, there is provided a use of an RBM3 protein, or an antigenically active fragment thereof, in the manufacture of a prognostic agent for establishing a prognosis for a mammalian subject having a breast cancer.

As an example, the prognostic agent of the above aspects may be an antibody or a fragment or a derivative thereof. For example, the prognostic agent may selectively bind to a molecular marker of prognostic relevance.

In the context of the present disclosure, a "prognostic agent" refers to an agent having at least one property being valuable in an establishment of a prognosis.

The inventors have found that the use, such as the identification and quantification, of an RBM3 protein prognostic value. Consequently, as a further aspect of the present invention, there is provided a use of an RBM3 protein as a prognostic marker, e.g. the use of an RBM3 protein as a prognostic marker for a cancer, such as a breast cancer.

In the context of the present disclosure, a "prognostic marker" refers to a product which presence is of value in an establishment of a prognosis.

In embodiments of the above use aspects, the RBM3 protein may for example comprise a sequence selected from: i) SEQ ID NO:1; and ii) a sequence which is at least 85 % identical to SEQ ID NO:1. In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1. Also, the RBM3 protein may for example comprise a sequence selected from: i) SEQ ID NO:2; and ii) a sequence which is at least 85 % identical to SEQ ID NO:2. In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

The inventors have found a previously unknown correlation between RBM3 protein and the prognosis for a patient suffering from a breast cancer. Further, the inventors have developed an affinity ligand with affinity for the RBM3 protein. However, the inventive scope is not limited to a single type of affinity ligand, and the inventive idea may be put into practice using any type of affinity ligand capable of selective interaction with an RBM3 protein.

Thus, as a further aspect of the present invention, the present invention provides an affinity ligand capable of selective interaction with an RBM3 protein. In one embodiment, the affinity ligand is an antibody or a fragment or a derivative thereof. Such an antibody, or fragment or derivative thereof, may for example be one that is obtainable by a process comprising a step of immunizing an animal, such as a rabbit, with a protein whose amino acid sequence comprises the sequence SEQ ID NO:1 or a sequence which is at least 85 % identical to SEQ ID NO:1, preferably the sequence SEQ ID NO:1. For example, the immunization process may comprise primary immunization with the protein in Freund's complete adjuvant. Also, the immunization process may further comprise boosting at least two times, in intervals of 2-6 weeks, with the protein in Freund's incomplete adjuvant. Processes for the production of antibodies or fragments or derivatives thereof against a given target are known in the art, and may be applied in connection with this aspect of the present invention. Any of those variants of the RBM3 protein (SEQ ID NO:2) or the antigenically active fragment thereof (SEQ ID NO:1) that are discussed above may, of course, be used in such a process for generating an antibody or a fragment or derivative thereof. The present disclosure further provides the above affinity ligand for establishing a prognosis for a mammalian subject having a breast cancer.

As a further aspect of the present disclosure, there is provided the use of the affinity ligand according to the above aspect as a prognostic agent. A preferred embodiment of this use is use of the affinity ligand as a prognostic agent for the prognosis of a cancer, such as a breast cancer. As a related aspect thereof, there is provided a use of the affinity ligand in the manufacture of a prognostic agent for the prognosis of a breast cancer.

The inventors have found that an endocrine treatment, such as tamoxifen treatment, appears to be particularly effective for hormone positive subjects having low levels of RBM3 protein.

Thus, as a further aspect of the present disclosure, there is provided an endocrine treatment product for use in the treatment of a mammalian subject having a breast cancer, wherein the subject is diagnosed as hormone receptor positive and RBM3 protein negative.

Further, as a related aspect thereof, there is provided a use of an endocrine treatment product in the manufacture of a medicament for treatment of a mammalian subject having a breast cancer, wherein the subject is diagnosed as hormone receptor positive and RBM3 protein negative.

In the context of the present disclosure, an "endocrine treatment product" refers to a compound or medicament for systemic treatment having anti-estrogenic effect. "Anti-estrogenic effect" is defined above.

The subject being diagnosed as "hormone receptor positive" refers to that the breast cancer from the subject has been found to be positive for a hormone receptor, such as ER or PR. The person skilled in the art knows how to determine whether a breast cancer is positive for a hormone receptor or not. For example, a commercially available kit for determining ER or PR status may be used for the determination. The commercially available kit may for example be ER/PR pharmDX (DakoCytomation) and the skilled person may use the kit according to the manufacturers instructions.

Further, for establishing an ER or PR diagnosis, the skilled person may turn to Allred et al (1998) Mod Pathol 11 (2), 155. Allred presents a total score (Allred score), and, for both ER and PR, an Allred score of higher than two is considered positive and an Allred score of two or lower is considered negative.

Alternatively, when classifying a sample as being hormone receptor positive or negative, such as ER+ or ER-, the skilled person may use 10 % positive cells as the cutoff, which is a recognized limit within the art.

The subject being diagnosed as "RBM3 protein low" refers to that a tumor of the subject has been found low for RBM3. For example, the subject may be considered RBM3 protein negative if a relevant biological sample from the subject has been found to contain an amount of RBM3 protein corresponding to a sample value being lower than a relevant reference value. Relevant samples and reference values are discussed above in connection with the method aspects. As a non-limiting example, a breast cancer subject may be considered as RBM3 protein low if a relevant sample such as a primary or secondary tumor sample from the subject contains an amount of RBM3 corresponding to NF ≤ 75 %.

In embodiments of the endocrine treatment product aspects, the endocrine treatment product may be selected from a selective estrogen receptor modulator (SERM), an aromatase, and a steroidal estrogen receptor antagonist. The SERM may be e.g. toremifene, raloxifene, droloxifene, arzoxifene or tamoxifene. The aromatase inhibitor may be e.g. anastrozole, letrozole och exemestane. Further, the steroidal estrogen receptor antagonist may be fulvestrant. As an example, the endocrine treatment product may be a SERM, such as a SERM selected from toremifene, raloxifene, droloxifene, arzoxifene or tamoxifene. For example, in embodiments of the endocrine treatment product aspects, the endocrine treatment may be tamoxifen.

In embodiments of the above aspects relating to the RBM3 protein or uses thereof, the affinity ligand or uses thereof, or the endocrine treatment product, the subject may be a human female and/or a premenopausal female. Further, in those aspects, the breast cancer may be HR+, such as ER+ or PR+, ER+, PR+, or ER+ and PR+. Also, in those aspects, the breast cancer may be a breast cancer previously classified as T1N0M0 according to the TNM staging system or a breast cancer previously classified as pT2 N0 M0, pT1 N1 M0 or pT2 N1 M0 according to the TNM staging system. These embodiments are discussed above in connection with the method aspects of the present disclosure.

In the context of the present invention, "specific" or "selective" interaction of e.g. an affinity ligand with its target or antigen means that the interaction is such that a distinction between specific and non-specific, or between selective and non-selective, interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high specificity does not necessarily require high affinity. Molecules with low affinity (in the molar range) for its counterpart have been shown to be as specific as molecules with much higher affinity. In the case of the present invention, a specific or selective interaction refers to the extent to which a particular method can be used to determine the presence and/or amount of a specific protein, the target protein or a fragment thereof, under given conditions in the presence of other proteins in a sample of a naturally occurring or processed biological fluid. In other words, specificity or selectivity is the capacity to distinguish between related proteins. Specific and selective are sometimes used interchangeably in the present description.

In the context of the present invention, a "mono-specific antibody" is one of a population of polyclonal antibodies which has been affinity purified on its own antigen, thereby separating such mono-specific antibodies from other antiserum proteins and non-specific antibodies. This affinity purification results in antibodies that bind selectively to its antigen. In the case of the present invention, the polyclonal antisera are purified by a two-step immunoaffinity based protocol to obtain mono-specific antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al (2005) Proteomics 5:4327-4337).

In the context of the inventive methods of present disclosure, "earlier obtained" refers to obtained before the inventive method is performed. Consequently, if a sample earlier obtained from a subject is provided in a method, the method does not involve obtaining the sample from the subject, i.e. the sample was previously obtained from the subject in a step separate from the method.

Furthermore, it has been found by the inventors that RBM3 protein is expressed in breast cancer tissue, and that the RBM3 protein expression is much lower, or absent, in most other normal and tumor tissues.

Thus, according to an alternative aspect of the present disclosure, there is provided a method for diagnosis of a breast cancer, comprising a step of detecting an RBM3 protein.

In one embodiment of the diagnosis aspect, the RBM3 detection is carried out *in vitro.*

In one embodiment of the diagnosis aspect, the amino acid sequence of the RBM3 protein comprises a sequence selected from:
i) SEQ ID NO:1; and
ii) a sequence which is at least 85 % identical to SEQ ID NO:1.

In another embodiment of the diagnosis aspect, the amino acid sequence of the RBM3 protein comprises a sequence selected from:
i) SEQ ID NO:2; and
ii) a sequence which is at least 85 % identical to SEQ ID NO:2.

In one embodiment of the diagnosis aspect, the method comprises the steps:
a. providing a sample from a mammalian subject suspected of having a breast cancer;
b. applying to the sample a detectable affinity ligand capable of selective interaction with the RBM3 protein to be detected, the application being performed under conditions that enable binding of the affinity ligand to RBM3 protein present in the sample;
c. removing non-bound affinity ligand; and
d. detecting any affinity ligand remaining in association with the sample.

In one embodiment, the sample of step a. is an earlier obtained sample.

In one embodiment, the sample of step a. is a tissue sample, such as a tissue sample derived from a breast of the mammalian subject.

Further embodiments and examples of the alternative aspect are apparent to the skilled person from the description of the previous aspects of the present disclosure.

### Brief description of the figures

Figure 1 shows the results of a survival analysis based on immunohistochemistry (IHC) staining of 151 subjects diagnosed with breast carcinoma in Cohort I. Figure 1 a shows overall survival (OS) in all patients. Figure 1b shows OS in ER+ patients. Figure 1c shows OS in HR+ patients. Tissue cores were scored for high or low RBM3 level, wherein a high RBM3 level was nuclear fraction (NF) > 25% (H), and a low RBM3 level was NF ≤ 25% (L).

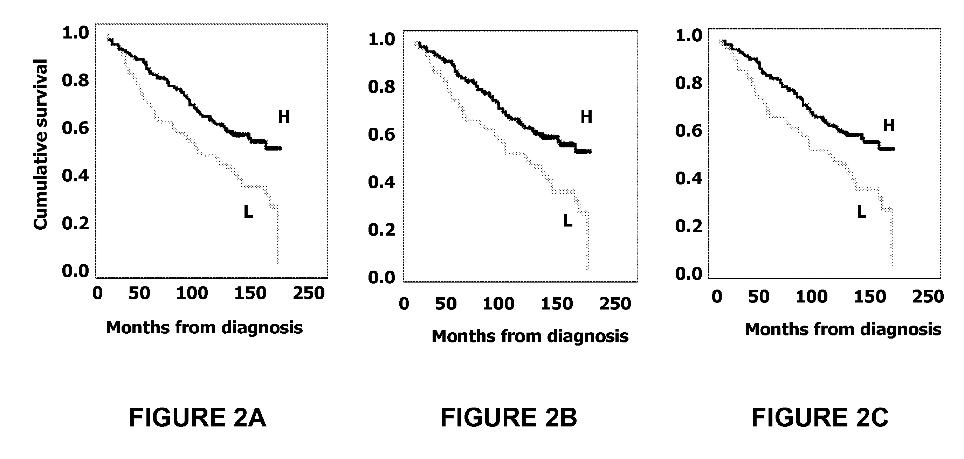

Figure 2 shows the results of a survival analysis based on IHC staining of 239 subjects diagnosed with breast carcinoma in Cohort II. Figure 2a shows OS in all patients. Figure 2b shows OS in ER+ patients. Figure 2c OS survival in HR+ patients. With regard to figures 2-6, tissue cores were scored for high or low RBM3 level, wherein a high RBM3 level was NF > 75% (H), and a low RBM3 level was NF ≤ 75% (L).

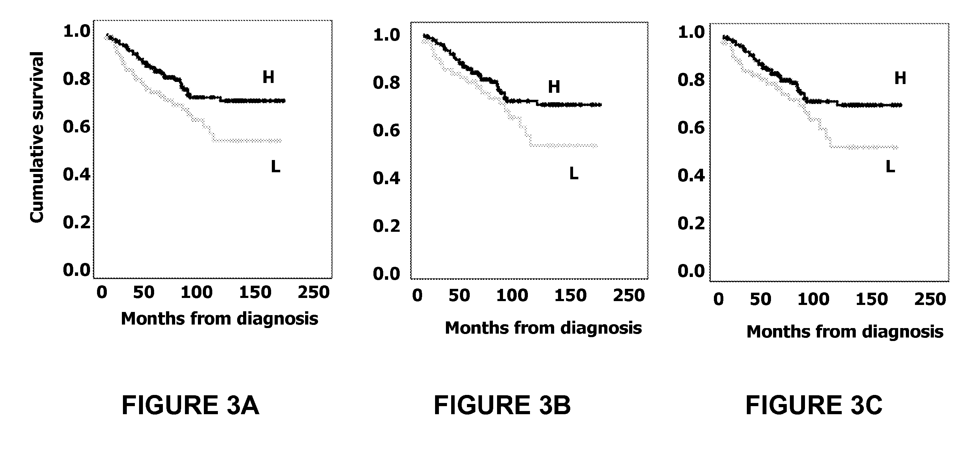

Figure 3 shows the results of a survival analysis based on IHC staining of 235 subjects diagnosed with breast carcinoma in Cohort II. Figure 3a shows recurrence free survival (RFS) in all patients. Figure 3b shows RFS in ER+ patients. Figure 3c shows RFS in HR+ patients.

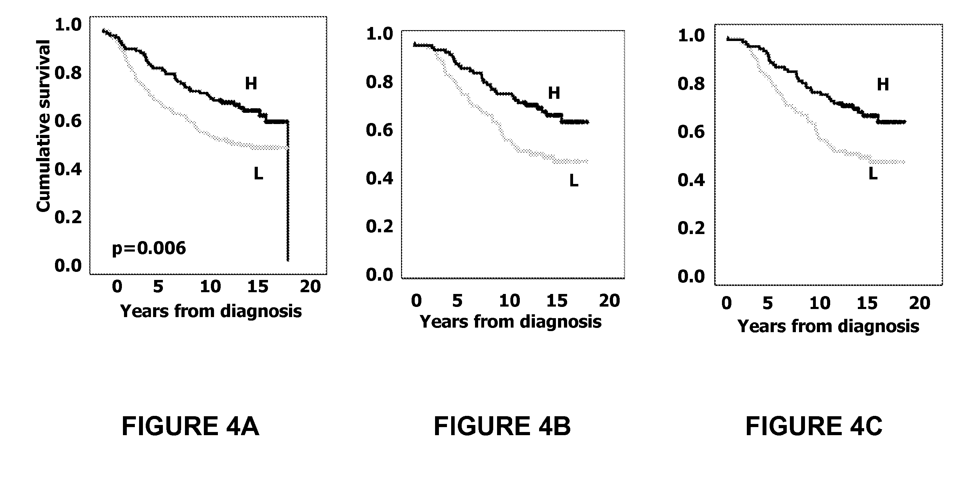

Figure 4 shows the results of a survival analysis based on IHC staining of 311 subjects diagnosed with breast carcinoma in Cohort III. Figure 4a shows OS in all patients. Figure 4b shows OS in ER+ patients. Figure 4c shows OS in HR+ patients.

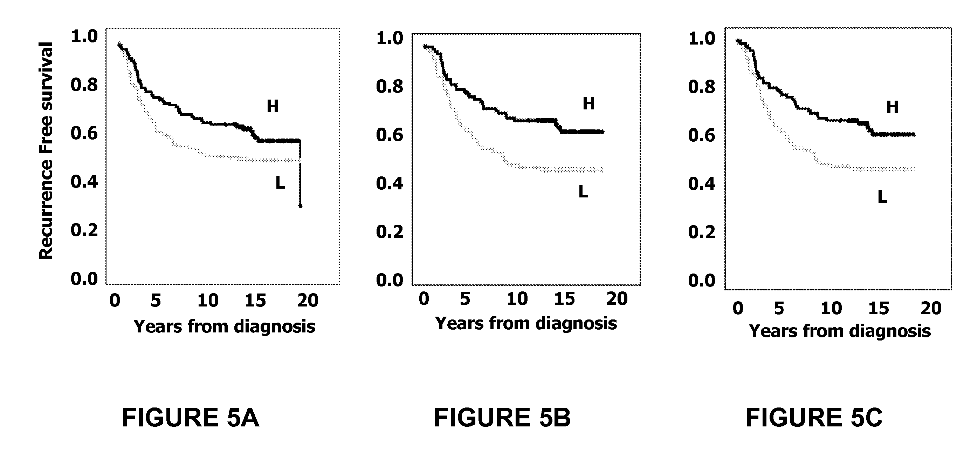

Figure 5 shows the results of a survival analysis based on IHC staining of 310 subjects diagnosed with breast carcinoma in Cohort III. Figure 5a shows RFS in all patients. Figure 5b shows RFS in ER+ patients. Figure 5c shows RFS in HR+ patients.

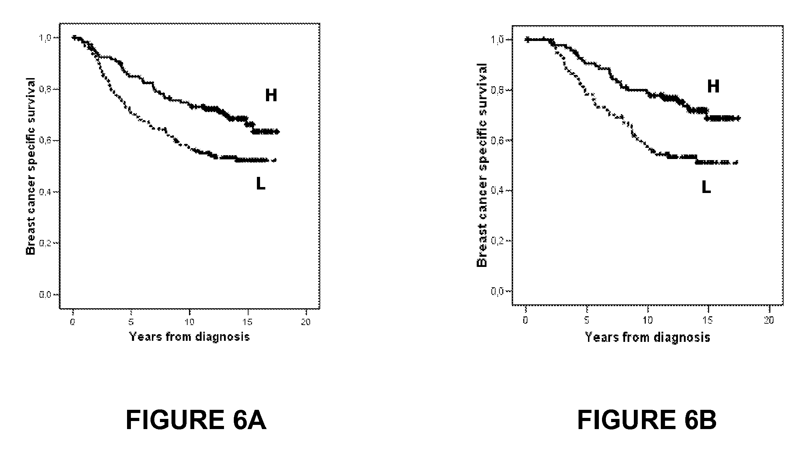

Figure 6 shows the results of a survival analysis based on IHC staining of 310 subjects diagnosed with breast carcinoma in Cohort III. Figure 6a shows breast cancer specific survival (BCSS) in all patients. Figure 6b shows BCSS in ER+ patients.
Figure 7 shows the results of a survival analysis based on IHC staining of 310 subjects diagnosed with breast carcinoma in Cohort III. Figure 7a shows RFS in ER+ patients with high RBM3 expression (NF > 75%). Figure 7b shows RFS in ER+ patients with low RBM3 expression (NF ≤ 75%). Further, "T" represents a group of subjects given adjuvant tamoxifen for 2 years, and "C" represents a control group not given tamoxifen.
Figure 8 shows the results of a survival analysis based on IHC staining of 310 subjects diagnosed with breast carcinoma in Cohort III. Figure 8a shows RFS in HR+ patients with high RBM3 expression (NF > 75%). Figure 8b shows RFS in HR+ patients with low RBM3 expression (NF ≤ 75%). Further, "T" represents a group of subjects given adjuvant tamoxifen for 2 years, and "C" represents a control group not given tamoxifen.

### Examples

### Generation of mono-specific antibodies against RBM3 and use thereof to detect RBM3 in normal and cancerous samples

### 1. Generation of antigen

### a) Materials and methods

A suitable fragment of the target protein encoded by the EnsEMBL Gene ID ENSG00000102317 was selected using bioinformatic tools with the human genome sequence as template (Lindskog M et al (2005) Biotechniques 38:723-727, EnsEMBL, www.ensembl.org). The fragment was used as template for the production of a 134 amino acid long fragment corresponding to amino acids 18-151 (SEQ ID NO:1) of the RBM3 protein (SEQ ID NO:2; EnsEMBL entry no. ENSP00000365946).

A fragment of the RBM3 gene transcript containing nucleotides 281-682 of EnsEMBL entry number ENST00000376755 (SEQ ID NO:3), was isolated by a Superscript^{™} One-Step RT-PCR amplification kit with Platinum® Taq (Invitrogen) and a human total RNA pool panel as template (Human Total RNA Panel IV, BD Biosciences Clontech). Flanking restriction sites NotI and AscI were introduced into the fragment through the PCR amplification primers, to allow in-frame cloning into the expression vector (forward primer: GACGAGCAGGCACTGGAAG, reverse primer: GTAATTTCCTCCTGAGTAGC). Then, the downstream primer was biotinylated to allow solid-phase cloning as previously described, and the resulting biotinylated PCR product was immobilized onto Dynabeads M280 Streptavidin (Dynal Biotech) (Larsson M et al (2000) J. Biotechnol. 80:143-157). The fragment was released from the solid support by Notl-Ascl digestion (New England Biolabs), ligated into the pAff8c vector (Larsson M *et al, supra*) in frame with a dual affinity tag consisting of a hexahistidyl tag for immobilized metal ion chromatography (IMAC) purification and an immunopotentiating albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into E. *coli* BL21 (DE3) cells (Novagen). The sequences of the clones were verified by dye-terminator cycle sequencing of plasmid DNA amplified using TempliPhi DNA sequencing amplification kit (GE Healthcare, Uppsala, Sweden) according to the manufacturer's recommendations.

BL21 (DE3) cells harboring the expression vector were inoculated in 100 ml 30 g/I tryptic soy broth (Merck KGaA) supplemented with 5 g/I yeast extract (Merck KGaA) and 50 mg/l kanamycin (Sigma-Aldrich) by addition of 1 ml of an overnight culture in the same culture medium. The cell culture was incubated in a 1 liter shake flask at 37 °C and 150 rpm until the optical density at 600 nm reached 0.5-1.5. Protein expression was then induced by addition of isopropyl-β-D-thiogalactopyranoside (Apollo Scientific) to a final concentration of 1 mM, and the incubation was continued overnight at 25 °C and 150 rpm. The cells were harvested by centrifugation at 2400 g, and the pellet was re-suspended in 5 ml lysis buffer (7 M guanidine hydrochloride, 47 mM Na₂HPO₄, 2.65 mM NaH₂PO₄, 10 mM Tris-HCl, 100 mM NaCl, 20 mM β-mercaptoethanol; pH = 8.0) and incubated for 2 hours at 37 °C and 150 rpm. After centrifugation at 35300 g, the supernatant containing the denatured and solubilized protein was collected.

The His₆-tagged fusion protein was purified by immobilized metal ion affinity chromatography (IMAC) on columns with 1 ml Talon® metal (Co²⁺) affinity resin (BD Biosciences Clontech) using an automated protein purification procedure (Steen J et al (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). Clarified cell lysates were then added to the column. Thereafter, the resin was washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, containing the antigen, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Vivascience AG). The protein concentration was determined using a bicinchoninic acid (BCA) micro assay protocol (Pierce) with a bovine serum albumin standard according to the manufacturer's recommendations. The protein quality was analyzed on a Bioanalyzer instrument using the Protein 50 or 200 assay (Agilent Technologies).

### b) Results

A gene fragment corresponding to nucleotides 281-682 of the long transcript (SEQ ID NO:3) of the RBM3 gene and encoding a peptide (SEQ ID NO:1) consisting of amino acids 18 to 151 of the target protein RBM3 (SEQ ID NO:2) was successfully isolated by RT-PCR from a human RNA pool using primers specific for the protein fragment. The 134 amino acid fragment (SEQ ID NO:1) of the target protein (SEQ ID NO:2) was designed to lack transmembrane regions to ensure efficient expression in *E. coli,* and to lack any signal peptide, since those are cleaved off in the mature protein. In addition, the protein fragment was designed to consist of a unique sequence with low homology with other human proteins, to minimize cross reactivity of generated affinity reagents, and to be of a suitable size to allow the formation of conformational epitopes and still allow efficient cloning and expression in bacterial systems.

A clone encoding the correct amino acid sequence was identified, and, upon expression in *E. coli,* a single protein of the correct size was produced and subsequently purified using immobilized metal ion chromatography. After dilution of the eluted sample to a final concentration of 1 M urea and concentration of the sample to 1 ml, the concentration of the protein fragment was determined to be 10.423 mg/ml and was 96.0 % pure according to purity analysis.

### 2. Generation of antibodies

### a) Materials and methods

The purified RBM3 fragment as obtained above was used as antigen to immunize a rabbit in accordance with the national guidelines (Swedish permit no. A 84-02). The rabbit was immunized intramuscularly with 200 µg of antigen in Freund's complete adjuvant as the primary immunization, and boosted three times in four week intervals with 100 µg antigen in Freund's incomplete adjuvant.

Antiserum from the immunized animal was purified by a three-step immunoaffinity based protocol (Agaton C et al (2004) J. Chromatogr. A 1043:33-40; Nilsson P et al (2005) Proteomics 5:4327-4337). In the first step, 7 ml of total antiserum was buffered with 10x PBS to a final concentration of 1x PBS (1.9 mM NaH₂PO₄, 8.1 mM Na₂HPO₄, 154 mM NaCl), filtered using a 0.45 µm pore-size filter (Acrodisc®, Life Science) and applied to an affinity column containing 5 ml N-hydroxysuccinimide-activated Sepharose^{™} 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragment. In the second step, the flow-through, depleted of antibodies against the dual affinity tag His₆-ABP, was loaded at a flow rate of 0.5 ml/min on a 1 ml Hi-Trap NHS-activated HP column (GE Healthcare) coupled with the RBM3 protein fragment used as antigen for immunization (SEQ ID NO:1). The His₆-ABP protein and the protein fragment antigen were coupled to the NHS activated matrix as recommended by the manufacturer. Unbound material was washed away with 1x PBST (1x PBS, 0.1 % Tween20, pH 7.25), and captured antibodies were eluted using a low pH glycine buffer (0.2 M glycine, 1 mM EGTA, pH 2.5). The eluted antibody fraction was collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen selective (mono-specific) antibodies (msAbs) were eluted with PBS buffer, supplemented with glycerol and NaN₃ to final concentrations of 40 % and 0.02 %, respectively, for long term storage at -20 °C (Nilsson P et al (2005) Proteomics 5:4327-4337).

The specificity and selectivity of the affinity purified antibody fraction were analyzed by binding analysis against the antigen itself and against 94 other human protein fragments in a protein array set-up (Nilsson P et al (2005) Proteomics 5:4327-4337). The protein fragments were diluted to 40 µg/ml in 0.1 M urea and 1x PBS (pH 7.4) and 50 µl of each were transferred to the wells of a 96-well spotting plate. The protein fragments were spotted in duplicate and immobilized onto epoxy slides (SuperEpoxy, TeleChem) using a pin-and-ring arrayer (Affymetrix 427). The slide was washed in 1x PBS (5 min) and the surface was then blocked (SuperBlock®, Pierce) for 30 minutes. An adhesive 16-well silicone mask (Schleicher & Schuell) was applied to the glass before the mono-specific antibodies were added (diluted 1:2000 in 1x PBST to appr. 50 ng/ml) and incubated on a shaker for 60 min. Affinity tag-specific IgY antibodies were co-incubated with the mono-specific antibodies in order to quantify the amount of protein in each spot. The slide was washed with 1x PBST and 1x PBS twice for 10 min each. Secondary antibodies (goat anti-rabbit antibody conjugated with Alexa 647 and goat anti-chicken antibody conjugated with Alexa 555, Molecular Probes) were diluted 1:60000 to 30 ng/ml in 1x PBST and incubated for 60 min. After the same washing procedure, as for the first incubation, the slide was spun dry and scanned (G2565BA array scanner, Agilent), thereafter images were quantified using image analysis software (GenePix 5.1, Axon Instruments).

In addition, the specificity and selectivity of the affinity-purified antibody were analyzed by Western blot. Western blot was performed by separation of total protein extracts from selected human cell lines on precast 10-20 % SDS-PAGE gradient gels (Bio-Rad Laboratories) under reducing conditions, followed by electro-transfer to PVDF membranes (Bio-Rad Laboratories) according to the manufacturer's recommendations. The membranes were blocked (5 % dry milk, 1x TBST; 0.1 M Tris-HCl, 0.5 M NaCl, 0.1 % Tween20) for 1 h at room temperature, incubated with the primary affinity purified antibody (diluted 1:500 in blocking buffer) and washed in TBST. The secondary HRP-conjugated antibody (swine anti-rabbit immunoglobulin/HRP, DakoCytomation) was diluted 1:3000 in blocking buffer and chemiluminescence detection was carried out using a Chemidoc^{™} CCD camera (Bio-Rad Laboratories) and SuperSignal® West Dura Extended Duration substrate (Pierce), according to the manufacturer's protocol.

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al (2004) J. Chromatogr. A 1043:33-40). Thus, a protein microarray analysis was performed to ensure that mono-specific polyclonal antibodies of high specificity had been generated by depletion of antibodies directed against the His₆-tag as well as of antibodies against the ABP-tag.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit msAb to its antigen on the array was detected with a fluorescently Alexa 647 labeled goat anti-rabbit antibody. Each protein fragment was spotted in duplicates. The protein array analysis shows that the affinity purified mono-specific antibody against RBM3 is highly selective to the correct protein fragment and has a very low background to all other protein fragments analyzed on the array.

The result of the Western blot analysis shows that the antibody specifically detects a single band of approximately 16 kDa in two breast tumor cell lines, T47D and MCF-7. The theoretical molecular weight of RBM3 is 16 kDa (as calculated from the RBM3 amino acid sequence SEQ ID NO:2), corresponding well to the result obtained.

### 3. Tissue profiling by immunohistochemistry

### a) Material and methods

In total, 576 paraffin cores containing human tissues were analyzed using the mono-specific antibody sample obtained in Examples, section 2. All tissues used as donor blocks for tissue microarray (TMA) production were selected from the archives at the Department of Pathology, University Hospital, Uppsala, in agreement with approval from the local ethical committee. All tissue sections used for TMA analysis were examined to determine diagnosis and to select representative areas in donor blocks. Normal tissue was defined as microscopically normal (non-neoplastic) and was most often selected from specimens collected from the vicinity of surgically removed tumors. Cancer tissue was reviewed for diagnosis and classification. All tissues were formalin fixated, paraffin embedded, and sectioned for diagnostic purposes.

The TMA production was performed essentially as previously described (Kononen J et al (1998) Nature Med. 4:844-847; Kallioniemi OP et al (2001) Hum. Mol. Genet. 10:657-662). Briefly, a hole was made in the recipient TMA block and a cylindrical core tissue sample from the donor block was acquired and deposited in the recipient TMA block. This was repeated in an automated tissue arrayer from Beecher Instrument (ATA-27, Beecher Instruments, Sun Prairie, CA, USA) until a complete TMA design was produced. TMA recipient blocks were baked at 42 °C for 2 h prior to sectioning.

The design of TMA:s was focused on obtaining samples from a large range of representative normal tissues, and on including representative cancer tissues. This has previously been described in detail in Kampf C et al (2004) Clin. Proteomics 1:285-300. In brief, samples from 48 normal tissues and from 20 of the most common cancer types affecting humans were selected. In total, eight different designs of TMA blocks, each containing 72 cores of tissue with 1 mm diameter, were produced. Two of the TMA:s represented normal tissues, corresponding to 48 different normal tissues in triplicates from different individuals. The remaining 6 TMA:s represented cancer tissue from 20 different types of cancer. For 17 of the 20 cancer types, 12 individually different tumors were sampled, and for the remaining 3 cancer types, 4 individually different tumors were sampled, all in duplicates from the same tumor. The TMA blocks were sectioned with 4 µm thickness using a waterfall microtome (Leica), and placed onto SuperFrost® (Roche Applied Science) glass slides for IHC analysis.

Automated IHC was performed as previously described (Kampf C et al (2004) Clin. Proteomics 1:285-300). In brief, the glass slides were incubated for 45 min in 60 °C, de-paraffinized in xylene (2 x 15 min) and hydrated in graded alcohols. For antigen retrieval, slides were immersed in TRS (Target Retrieval Solution, pH 6.0, DakoCytomation) and boiled for 4 min at 125 °C in a Decloaking chamber® (Biocare Medical). Slides were placed in the Autostainer® (DakoCytomation) and endogenous peroxidase was initially blocked with H₂O₂ (DakoCytomation). The slides were incubated for 30 min at room temperature with the primary antibody obtained as in Examples, Section 2, followed by incubation for 30 min at room temperature with goat anti-rabbit peroxidase conjugated Envision@. Between all steps, slides were rinsed in wash buffer (DakoCytomation). Finally, diaminobenzidine (DakoCytomation) was used as chromogen and Harris hematoxylin (Sigma-Aldrich) was used for counterstaining. The slides were mounted with Pertex® (Histolab).

All immunohistochemically stained sections from the eight different TMA:s were scanned using a ScanScope T2 automated slide-scanning systems (Aperio Technologies). In order to represent the total content of the eight TMA:s, 576 digital images were generated. Scanning was performed at 20 times magnification. Digital images were separated and extracted as individual tagged image file format (TIFF) files for storage of original data. In order to be able to handle the images in a web-based annotation system, the individual images were compressed from TIFF format into JPEG format. All images of immunohistochemically stained tissue were manually evaluated under the microscope and annotated by a certified pathologist or by specially educated personnel, subsequently verified by a pathologist.

Annotation of each different normal and cancer tissue was performed using a simplified scheme for classification of IHC outcome. Each tissue was examined for representativity and immunoreactivity. The different tissue specific cell types included in each normal tissue type were annotated. For each cancer, tumor cells and stroma were annotated. Basic annotation parameters included an evaluation of i) subcellular localization (nuclear and/or cytoplasmic/membranous), ii) staining intensity (SI) and iii) fraction of stained cells (FSC). Staining intensity was subjectively evaluated in accordance to standards used in clinical histo-pathological diagnostics and outcome was classified as: absent = no immunoreactivity, weak = faint immunoreactivity, moderate = medium immunoreactivity or strong = distinct and strong immunoreactivity. The fraction of stained cells was estimated and classified as < 2 %, 2 - 25 %, > 25 - 75 % or > 75 % immunoreactive cells of the relevant cell population. Based on both the intensity and fraction of immunoreactive cells, a "staining score" was given for each tissue sample: 0 = negative, 1 = weak, 2 = moderate and 3 = strong. N.R. means that no representative tissues were present. In detail, the staining score was given according to the following criteria: 0 was given if SI = absent or weak and FSC ≤ 25%; 1 was given if SI = weak and FSC > 25% or if SI = moderate and FSC ≤ 25%; 2 was given if SI = moderate and FSC > 25% or if SI = strong and FSC ≤ 25% and SI = moderate; and finally 3 was given if SI = strong and FSC > 25%. See also table 1. The skilled artisan will recognize that this procedure is similar to a calculation of an Allred score, see e.g. Allred *et al* (1998) Mod Pathol 11 (2), 155.

| **Table 1:** Staining score | | |
|---|---|---|
| **Staining score** | **Staining intensity** | **Fraction of stained cells** |
| 0 | absent | < 2% |
| 0 | absent | 2 - 25% |
| 0 | absent | > 25 - 75% |
| 0 | absent | > 75% |
| 0 | weak | < 2% |
| 0 | weak | 2 - 25% |
| 1 | weak | > 25 - 75% |
| 1 | weak | > 75% |
| 1 | moderate | < 2% |
| 1 | moderate | 2 - 25% |
| 2 | moderate | > 25 - 75% |
| 2 | moderate | > 75% |
| 2 | strong | < 2% |
| 2 | strong | 2 - 25% |
| 3 | strong | > 25 - 75% |
| 3 | strong | > 75% |

### b) Results

The results from tissue profiling with the mono-specific antibody generated towards a recombinant protein fragment of the human target protein RBM3 obtained as in Examples, Section 2 showed a particular immunoreactivity in several normal tissues (Table 2) and cancer tissues. Immunoreactivity was observed in cytoplasm with the exception of spermatogonia cells of testis (Table 2), which displayed a distinct nuclear staining.

Table 2 shows the RBM3 protein expression pattern in normal human tissues. Using IHC and TMA technology, 144 spots (1 mm in diameter) representing 48 different types of normal tissue were screened for expression of RBM3. A weak immunoreactivity (staining score 1) was observed in glandular cells of breast epididymis and parathyroid gland. A weak expression was also observed in lung, placenta, skeletal muscle, skin and thyroid. No other cells and tissues showed positive immunoreactivity (staining score 0). In a few cases no representative tissue (N.R.) were observed.

| **Table 2:** Expression pattern of RBM3 in normal tissues | | |
|---|---|---|
| **Tissue type** | **Cell type** | **Staining score** |
| **Adrenal gland** | cortical cells | 0 |
| | medullar cells | N.R. |
| **Appendix** | glandular cells | 0 |
| | lymphoid tissue | 0 |
| **Bone marrow** | bone marrow poetic cells | 0 |
| **Breast** | glandular cells | 1 |
| **Bronchus** | surface epithelial cells | 0 |
| **Cerebellum** | cells in granular layer | 0 |
| | cells in molecular layer | 0 |
| | purkinje cells | 0 |
| **Cerebral cortex** | neuronal cells | 0 |
| | non-neuronal cells | 0 |
| **Cervix, uterine** | glandular cells | 0 |
| | surface epithelial cells (squamous) | 0 |
| **Colon** | glandular cells | 0 |
| **Duodenum** | glandular cells | 0 |
| **Endometrium 1** | cells in endometrial stroma/ECM | 0 |
| | cells in myometrium/ECM | 0 |
| | glandular cells | 0 |
| **Endometrium 2** | cells in endometrial stroma/ECM | 0 |
| | cells in myometrium/ECM | 0 |
| | glandular cells | 0 |
| **Epididymis** | glandular cells | 1 |
| **Esophagus** | surface epithelial cells | 0 |
| **Fallopian tube** | glandular cells | 0 |
| **Gall bladder** | glandular cells | 0 |
| **Heart muscle** | myocytes | 0 |
| **Hippocampus** | neuronal cells | 0 |
| | non-neuronal cells | 0 |
| **Kidney** | cells in glomeruli | 0 |
| | cells in tubuli | 0 |
| **Lateral ventricle** | neuronal cells | 0 |
| | non-neuronal cells | 0 |
| **Liver** | bile duct cells | 0 |
| | hepatocytes | 0 |
| **Lung** | alveolar cells | 0 |
| | macrophages | 1 |
| **Lymph node** | follicle cells (cortex) | 0 |
| | non-follicle cells (paracortex) | 0 |
| **Nasopharynx** | surface epithelial cells | 0 |
| **Oral mucosa** | surface epithelial cells | 0 |
| **Ovary** | follicle cells | N.R. |
| | ovarian stromal cells | 0 |
| **Pancreas** | exocrine pancreas | 0 |
| | islet cells | 0 |
| **Parathyroid gland** | glandular cells | 1 |
| **Placenta** | decidual cells | 0 |
| | trophoblastic cells | 1 |
| **Prostate** | glandular cells | 0 |
| **Rectum** | glandular cells | 0 |
| **Salivary gland** | glandular cells | 0 |
| **Seminal vescicle** | glandular cells | 0 |
| **Skeletal muscle** | myocytes | 1 |
| **Skin** | adnexal cells | N.R. |
| | epidermal cells | 1 |
| **Small intestine** | glandular cells | 0 |
| **Smooth muscle** | smooth muscle cells | 0 |
| **Soft tissue 1** | mesenchymal cells | 0 |
| **Soft tissue 2** | mesenchymal cells | 0 |
| **Spleen** | cells in red pulp | 0 |
| | cells in white pulp | 0 |
| **Stomach 1** | glandular cells | 0 |
| **Stomach 2** | glandular cells | 0 |
| **Testis** | cells in ductus seminiferus | 1 |
| | leydig cells | 0 |
| **Thyroid gland** | glandular cells | 0 |
| **Tonsil** | follicle cells (cortex) | 0 |
| | non-follicle cells (paracortex) | 0 |
| | surface epithelial cells | 1 |
| **Urinary bladder** | surface epithelial cells | 0 |
| **Vagina** | surface epithelial cells | 0 |
| **Vulva/anal skin** | surface epithelial cells | 0 |

RBM3 protein expression was further evaluated in tissue samples from various cancer types. Table 3 shows the level of RBM3 expression in 12 different breast carcinoma tissues samples. All of these samples showed representative tissue and ten showed positivity, i.e. medium or weak immunoreactivity. RBM3 expression was observed in both nuclei and cytoplasm.

| **Table 3:** Expression pattern of RBM3 | | | | | | | | | | | | |
|---|---|---|---|---|---|---|---|---|---|---|---|---|
| Tissue sample | 1 | 2 | 3 | 4 | 5 | 6 | 7 | 8 | 9 | 10 | 11 | 12 |
| Staining score | 2 | 2 | 1 | 1 | 1 | 1 | 1 | 1 | 1 | 1 | 0 | 0 |

### 4. Breast cancer TMA (Phase A) (Cohort I)

### a) Material and methods

Archival formalin-fixed paraffin-embedded tissue from 179 patients diagnosed with primary invasive breast cancer 2000 and 2002 was collected from the Department of Pathology, Malmö University Hospital, Sweden. The median age of the patients was 65 (range 35-97) years. After a median follow-up of 52 months (range 4-63), 37 patients were dead and 142 alive. Treatment data was not available for this cohort. Information regarding the date of death was obtained from the regional cause-of-death registries for all patients. Ethical permission was obtained from the Local Ethics Committee.

All 179 cases of breast cancer were histopathologically reevaluated on slides stained with hematoxylin and eosin. TMA:s were then constructed by sampling 2 x 1.0 mm cores per case from areas representative of invasive cancer, using a manual arraying device (MTA-1, Beecher Inc, Sun Prairie, WI, USA). The TMA:s were prepared and automated IHC was performed as described in section 3 above, using the RBM3 antibody prepared as described in section 2 above.

For statistical analyses, categories were based on nuclear fraction (NF), corresponding to the percentage of tumor cells in a sample that exhibits a positive staining in the nucleus. As with "the fraction of stained cells" in Examples, Section 3 above, NF was classified as < 2 %, 2-25 %, > 25 - 75 % or > 75 %. Two categories were defined: "RBM3 protein low" corresponding to NF ≤ 25%; and "RBM3 protein high" corresponding to NF > 25%.

This classification of samples was used for overall survival (OS) analysis according to the Kaplan-Meier estimator, and the log-rank test was used to compare survival in different strata. All statistical tests were two-sided, and p-values of < 0.05 % were considered significant. All calculations were made with the statistical package SPSS 12.0 (SPSS Inc. Illinois, USA).

### b) Results

Immunohistochemistry analysis of RBM3 expression could be performed on 151 tumors. The remaining cores either did not contain invasive cancer or had been lost during histoprocessing. Of the 151 analyzed tumors, 132 were ER+, and 133 were hormone receptor positive (HR+) (i.e. ER+ and/or PR+). IHC staining of ER and PR had been performed previously. In line with current clinical praxis, a cut-off at 10 % positive nuclei was used to define hormone receptor positivity. Analysis of overall survival (OS) based on high or low RBM3 protein levels in all patients, ER+ patients or HR+ patients are presented in fig. 1a-c, respectively. From these analyses, a better OS was observed for RBM3 protein high patients when analyzing the whole group, ER+ patients only or HR+ patients only. The results indicate a prognostic value for RBM3.

### 5. Consecutive cohort TMA (Cohort II)

### a) Material and methods

Archival formalin-fixed paraffin-embedded tissue from 512 patients diagnosed with primary breast cancer between 1988 and 1992 was collected from the Department of Pathology, Malmö University Hospital, Sweden. The median age of patients was 64.5 (range 27-96) years and median follow-up time was 106 months (0-207). Follow up data regarding RFS and death was available for 507 patients. Information regarding the date of death was obtained from the regional cause-of-death registries for all patients. Ethical permission was obtained from the Local Ethics Committee.

All 512 cases of breast cancer were histopathologically reevaluated on slides stained with hematoxylin and eosin. TMA:s were constructed by sampling 2 x 0.6 mm cores per case from areas representative of invasive cancer, using an automated arraying device (ATA-27, Beecher Inc, Sun Prairie, WI, USA). The TMA:s were prepared and automated IHC was performed as described in section 3 above, using the RBM3 antibody prepared as described in section 2 above.

For statistical analyses, categories were based on nuclear fraction (NF), corresponding to the percentage of tumor cells in a sample that exhibits a positive staining in the nucleus. As with "the fraction of stained cells" in Examples, Section 3 above, NF was classified as < 2 %, 2 - 25 %, > 25 - 75 % or > 75 %.

Based on the survival trends for all different strata, a dichotomized variable were constructed for further statistical analyses, defining "RBM3 protein high" as NF > 75 % and "RBM3 protein low" as NF ≤ 75 %. This classification of samples was used for OS and RFS analysis according to the Kaplan-Meier estimator, and the log-rank test was used to compare survival in different strata. All statistical tests were two-sided, and p-values of < 0.05 % were considered significant. All calculations were made with the statistical package SPSS 12.0 (SPSS Inc. Illinois, USA).

### b) Results

For the present study, IHC analysis of RBM3 expression could be performed on totally 239 tumors. The remaining cores either did not contain invasive cancer or had been lost during histoprocessing. Of the 239 analyzed tumors, 212 were HR+, and 210 were ER+. IHC staining of ER and PR had been performed previously. In line with current clinical praxis, a cut-off at 10 % positive nuclei was used to define hormone receptor positivity. Analysis of OS based on high or low RBM3 protein levels in all patients, HR+ patients or ER+ patients only are presented in fig 2a-c. From these analyses, a significantly better OS is observed for the RBM3 protein high category, than for the RBM3 protein low category, in all three patient groups. Analyses of RFS based on high or low RBM3 protein levels in the same three patients groups revealed a similar significant result (Fig. 3a-c).

Taken together, the results from the OS and RFS analyses indicate a prognostic value for a high RBM3 level in all patients, and specifically HR+ patients and the subgroup of ER+ only patients.

### 6. Randomized premenopausal cohort TMA (Cohort III)

### a) Material and methods

Immunohistochemistry analyses were performed on TMAs representing 500 tumors from 564 premenopausal patients enrolled in a controlled, randomized tamoxifen trial of stage II (pT2 NO MO, pT1 N1 MO or pT2 N1 M0) primary breast carcinoma. The patients had been included in the trial between 1986-1991 irrespective of hormone receptor status and 276 patients had been allocated to two years of adjuvant tamoxifen and 288 to control. Median age at diagnosis was 45 (25-57) years. Median follow-up was 13.9 years and less than 2% of the patients had received adjuvant chemotherapy. The study design is described in detail elsewhere (Rydén L et al, Eur J Cancer, 2005, 41 (2): 256-64). Ethical permission was obtained from the Local Ethics Committees at Lund and Linköping Universities.

All cases had been histopathologically re-evaluated on hematoxylin and eosin stained slides. TMA:s were constructed by sampling 2 x 0.6 mm cores per case from areas representative of invasive cancer, using an automated arraying device (ATA-27, Beecher Inc, Sun Prairie, WI, USA). The TMA:s were prepared and automated IHC was performed as described in section 3 above, using the RBM3 antibody prepared as described in section 2 above. IHC staining of ER and PR had been performed previously. In line with current clinical praxis, a cut-off at 10 % positive nuclei was used to define hormone receptor positivity.

For statistical analyses, categories were based on nuclear fraction (NF), corresponding to the percentage of tumor cells in a sample that exhibits a positive staining in the nucleus. As with "the fraction of stained cells" in Examples, Section 3 above, NF was classified as < 2%, 2 - 25 %, > 25 - 75 % or > 75 %. Two categories were defined: "RBM3 protein high" corresponding to NF > 75%; and "RBM3 protein low" corresponding to NF ≤ 75%. This classification of samples was used for OS, RFS and breast cancer specific survival (BCSS) analysis according to the Kaplan-Meier estimator, and the log-rank test was used to compare survival in different strata. In addition, Cox uni- and multivariate analysis of RFS and OS was analyzed in different patient strata. Multivariate analysis included adjustment for NHG (I-II vs III), nodal status 0 vs 1 and tumor size (</>20 mm). Also an adjustment for treatment was done. All statistical tests were two-sided, and p-values of < 0.05 % were considered significant. All calculations were made with the statistical package SPSS 12.0 (SPSS Inc. Illinois, USA).

### b) Results

Immunohistochemistry analysis of RBM3 expression could be performed on 311 of 500 tumor samples. The remaining cores either did not contain invasive cancer or had been lost during histoprocessing. Of the 311 analyzed tumors, 196 samples were ER+ and 205 were HR+ (ER+ and HR+ were defined as above). Analyses of OS and RFS based on high or low RBM3 level in all patients, ER+ patients and HR+ patients are presented in fig 4a-c and 5a-c. Similar to the consecutive cohort, a high fraction (>75%) of RBM3 positive nuclei was associated with significantly better OS and RFS. In multivariate analysis, when adjusted for the prognostic factors described above, and tamoxifen treatment, a similar trend showing a better prognosis for RBM3 protein high patients was observed, and for patients being ER+ or HR+, the prognostic value was significant.

In addition, analysis of BCSS based on high or low RBM3 protein levels on all patients or ER+ patients revealed a similar significant result (Fig. 6a-b).

Taken together, a high RBM3 level is significantly positive for OS, RFS and BCSS and the results indicate that the prognostic value of RBM3 is retained when adjusted for established prognostic parameters and even treatment.

If the parameters were changed, and the RFS analysis was based on tamoxifen treatment in ER+ patients, the result revealed that tamoxifen treatment had less impact on RFS of RBM3 protein high patients than RFS of RBM3 protein low patients (Fig. 7a and 7b). Similar results were also obtained for HR+ patients (Fig. 8a and 8b).

Consequently, RBM3 protein high patients appeared to benefit less from Tamoxifen treatment. Further, independent of Tamoxifen treatment, the RFS is generally better for RBM3 protein high patients than for RBM3 protein low patients.

Breast cancer is a truly heterogeneous disease and there is still a great need for additional biomarkers in order to better identify prognostic subgroups and tailor optimal individual treatment regimens. It is, for instance, widely believed that some patients with node negative disease and tumors < 20mm (T1N0M0) are over-treated with the prevailing approach to breast cancer management. Thus, there is a strong need for prognostic markers in order to select patients from this subgroup that do not need adjuvant polychemotherapy, while others may be under-treated.

According to current oncology protocols in Sweden, T1N0M0 patients with HR+ NHG III tumors should receive adjuvant polychemotherapy and patients with HR+ T1N0M0 NHG II-III tumors ≥10 mm, are offered adjuvant endocrine treatment. The prognostic value of RBM3 was independent of established prognostic parameters, including NHG, among HR+ tumors in both extended cohorts. The findings from the premenopausal randomized trial further indicate that this prognostic value seems to be independent of tamoxifen treatment and less than 2% of the patients had received adjuvant polychemotherapy. In the light of this, RBM3 expression is a promising marker for selecting T1N0M0 patients that do not need any adjuvant treatment at all, irrespective of histological grade, not least in premenopausal women for whom aromatase inhibitors are not an ideal alternative to tamoxifen.

### Establishment of a prognosis for a breast cancer patient

### 7. A non-limiting example

A breast cancer patient can present symptoms or signs such as a palpable lump/tumor, secretion from the mamilla or skin deformities. A proportion of breast cancers, generally without symptoms, are also detected by screening mammography.

Following the establishment of a breast cancer diagnosis in a patient, a biopsy of the suspected tumor tissue is performed to obtain a tumor tissue sample. A biopsy is the removal of a selected physical piece of tissue from the suspected tumor. Further, for the provision of a reference sample showing a weak RBM3 protein staining, a sample is taken from archival material comprising tissue having low, or essentially lacking, RBM3 protein expression. Such archival tissue may for example be breast carcinoma tissue having a pre-established low RBM3 protein expression level or an appropriate tissue having a staining score of 0 in Table 2. Further, for the provision of a reference sample showing a high RBM3 protein staining, a sample is taken from archival material comprising tissue having high RBM3 protein expression, such as breast carcinoma tissue having a pre-established high RBM3 protein expression level.

The sample material is fixated in buffered formalin and histo-processed in order to obtain a thin sections (4 µm) of the of the sample material.

Immunohistochemistry is performed as described in Examples, section 3. One or more sample sections from each sample are mounted on glass slides that are incubated for 45 min in 60 °C, de-paraffinized in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple sample sections may be to increase the accuracy of the results.

A primary RBM3 specific antibody is added to the slides and incubated for 30 min in room temperature, followed by a 30 min incubation in room temperature with a labeled secondary antibody; e.g. goat-anti-rabbit peroxidase conjugated Envision®. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab) mounting media.

As a tool to validate the staining procedure, two control cell-lines may be used; e.g. one slide with cells expressing RBM3 (positive cell line) and one slide having cells with indistinct weak or no RBM3 expression (negative cell line). The skilled artisan understands how to provide such cell lines, for example guided by the disclosure of Rhodes et al. (2006) The biomedical scientist, p 515-520. The control-line slides may be simultaneously stained in the same procedure as the breast cancer slides, i.e. incubated with the same primary and secondary antibodies.

For example, the breast cancer tumor slides, the staining reference slides (derived from the reference samples), and optionally, the slides with control cell-lines, may be scanned in a light microscope using a ScanScope T2 automated slide scanning system (Aperio Technologies) at x20 magnification.

If control cell-lines are used, these are inspected to validate the staining procedure. If the cell-lines display staining results outside acceptable criteria, e.g. staining artifacts recognized by the skilled artisan, the staining of the biopsy samples is considered as invalid and the whole staining procedure is repeated with new slides. If the positive and negative cell-lines display strong staining intensity and indistinct weak or no staining intensity, respectively, the staining is considered as valid.

The stained sample slide(s) from the tumor tissue biopsy are evaluated manually by visual inspection in accordance to standards used in clinical histo-pathological diagnostics, and the immunoreactivity of the breast cancer slides are graded as described in Examples, section 4. That is, the nuclear fraction (NF) is determined, corresponding to the percentage of cells in the sample that exhibits a positive staining in the nucleus. The nuclear fraction of the sample slides are subjectively classified as < 2 %, 2 - 25 %, > 25 - 75 % or > 75 %.

The person performing the evaluation and grading is aided by visual inspection of the stained reference slides having high and low amount of, or no, RBM3 protein expression, respectively: the reference slide having high RBM3 protein provides a "positive reference", and the reference slide having a low amount of, or no, RBM3 protein expression provides a "negative reference".

The sample value(s), i.e. the NF(s), of the sample slide(s) from the tumor tissue biopsy are then compared to a reference value. The reference value may for example be a NF of 75 %. If the sample value(s) are higher than the reference value, a conclusion is drawn that the prognosis is better than a reference prognosis being associated with the reference value. For example, if the reference value is a NF of 75 %, which may be associated with a probability of five-year recurrence free survival of 63 %, the conclusion may be that the prognosis for the patient is a probability of five-year recurrence free survival of over 63 %. The prognosis may then form a basis for further decisions relating to the treatment, or non-treatment, of the patient.

## Claims

1. Method for determining whether a prognosis for a mammalian subject having a breast cancer is better than a reference prognosis, comprising the steps of:
a) providing a sample earlier obtained from the subject;
b) evaluating the amount of RBM3 protein present in at least part of said sample, and determining a sample value corresponding to said evaluated amount;
c) comparing the sample value obtained in step b) with a reference value associated with said reference prognosis; and, if said sample value is higher than said reference value,
d) concluding that the prognosis for said subject is better than said reference prognosis.

2. Method for determining whether a subject having a breast cancer is not in need of an adjuvant breast cancer treatment:
a) providing an earlier obtained sample from the subject;
b) evaluating the amount of RBM3 protein present in at least part of said sample, and determining a sample value corresponding to said evaluated amount;
c) comparing the sample value obtained in step b) with a reference value; and, if said sample value is higher than said reference value,
d) concluding that said subject is not in need of said adjuvant breast cancer treatment.

3. Non-treatment strategy method for a subject having a breast cancer, comprising:
a) providing an earlier obtained sample from the subject;
b) evaluating the amount of RBM3 protein present in at least part of said sample, and determining a sample value corresponding to said evaluated amount;
c) comparing the sample value obtained in step b) with a reference value; and, if said sample value is higher than said reference value,
d) refraining from treating said subject with an adjuvant breast cancer treatment.

4. Method of treatment of a subject having a breast cancer, comprising:
a) providing a sample from the subject;
b) evaluating the amount of RBM3 protein present in at least part of said sample, and determining a sample value corresponding to said evaluated amount;
c) comparing the sample value obtained in step b) with a reference value; and, if said sample value is equal to or lower than said reference value,
d) treating said subject with an adjuvant breast cancer treatment.

5. Method according to any one of claims 1-4, wherein said sample is a tissue sample.

6. Method according to claim 5, wherein said tissue sample is a breast tumor sample.

7. Method according to claim 6, wherein the evaluation of step b) is limited to evaluating the amount of RBM3 protein expression in the nucleus of tumor cells of said tissue sample.

8. Method according to any preceding claim, wherein said subject is a human female.

9. Method according any preceding claim, wherein said subject is a premenopausal female.

10. Method according any preceding claim, wherein said breast cancer is hormone receptor positive.

11. Method according any preceding claim, wherein said breast cancer is previously classified as T1N0M0 according to the TNM staging system.

12. Method according to any one of claims 1-10, wherein said breast cancer is previously classified as pT2 N0 M0, pT1 N1 M0 or pT2 N1 M0 according to the TNM staging system.

13. Method according any preceding claim, wherein said prognosis is a probability of survival.

14. Method according to any preceding claim, wherein said reference value is a value corresponding to a predetermined amount of RBM3 protein in a reference sample.

15. Method according to any preceding claim, wherein said reference value is a nuclear fraction, a nuclear intensity, or a function of a nuclear fraction and a nuclear intensity.

16. Method according to claim 15, wherein said reference value is a nuclear fraction.

17. Method according to any preceding claim, wherein said reference value is a nuclear fraction of 10 - 90 % RBM3 protein positive cells.

18. Method according claim 17, wherein said reference value is a nuclear fraction of 20 - 80 % RBM3 protein positive cells.

19. Method according to any preceding claim, wherein the amino acid sequence of the RBM3 protein comprises a sequence selected from:
i) SEQ ID NO:1; and
ii) a sequence which is at least 85 % identical to SEQ ID NO:1.

20. Method according to any preceding claim, wherein the amino acid sequence of the RBM3 protein comprises a sequence selected from:
i) SEQ ID NO:2; and
ii) a sequence which is at least 85 % identical to SEQ ID NO:2.

21. Method according to any preceding claim, wherein step b) comprises:
b1) applying to the sample a quantifiable affinity ligand capable of selective interaction with the RBM3 protein to be quantified, said application being performed under conditions that enable binding of the affinity ligand to any RBM3 protein present in the sample;
b2) removing non-bound affinity ligand; and
b3) quantifying any affinity ligand remaining in association with the sample to evaluate said amount.

22. Method according to claim 21, wherein the quantifiable affinity ligand is selected from the group consisting of antibodies, fragments thereof and derivatives thereof.

23. Method according to any one of claims 21-22, wherein the quantifiable affinity ligand comprises a label selected from the group consisting of fluorescent dyes and metals, chromophoric dyes, chemiluminescent compounds and bioluminescent proteins, enzymes, radioisotopes, particles and quantum dots.

24. Method according to any one of claims 21-23, in which said quantifiable affinity ligand is detected using a secondary affinity ligand capable of recognizing the quantifiable affinity ligand.

25. Method according to claim 24, in which said secondary affinity ligand capable of recognizing the quantifiable affinity ligand comprises a label selected from the group consisting of fluorescent dyes and metals, chromophoric dyes, chemiluminescent compounds and bioluminescent proteins, enzymes, radioisotopes, particles and quantum dots.

26. Kit for carrying out the method according to any preceding claim, which comprises
a) a quantifiable affinity ligand capable of selective interaction with an RBM3 protein; and
b) reagents necessary for quantifying the amount of the affinity ligand.

27. Kit according to claim 26, in which the quantifiable affinity ligand is selected from the group consisting of antibodies, fragments thereof and derivatives thereof.

28. Kit according to claim 27, in which the quantifiable affinity ligand is obtainable by a process comprising a step of immunizing an animal with a protein whose amino acid sequence comprises the sequence SEQ ID N0:1.

29. Kit according to any one of claims 26-28, in which the quantifiable affinity ligand comprises a label selected from the group consisting of fluorescent dyes and metals, chromophoric dyes, chemiluminescent compounds and bioluminescent proteins, enzymes, radioisotopes, particles and quantum dots.

30. Kit according to any one of claims 26-29, in which said reagents necessary for quantifying the amount of the affinity ligand comprise a secondary affinity ligand capable of recognizing the quantifiable affinity ligand.

31. Kit according to claim 30, in which said secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

32. Kit according to any one of claims 26-31, further comprising a reference sample comprising RBM3 protein for provision of a reference value.

33. Kit according to claim 32, wherein the reference sample contains an amount of RBM3 protein corresponding to a nuclear fraction of 10 - 90 % RBM3 protein positive cells.

34. Kit according to claim 33, wherein the reference sample contains an amount of RBM3 protein corresponding to a nuclear fraction of 20 - 80 % RBM3 protein positive cells.

35. Kit according to any one of claims 30-34, wherein the reference sample is a tissue sample.

36. RBM3 protein or an antigenically active fragment thereof for manufacture of a prognostic agent for establishing a prognosis for a mammalian subject having a breast cancer.

37. RBM3 protein according to claim 36, wherein the amino acid sequence of the RBM3 protein comprises a sequence selected from:
i) SEQ ID NO:1; and
ii) a sequence which is at least 85 % identical to SEQ ID NO:1.

38. RBM3 protein according to claim 36, wherein the amino acid sequence of the RBM3 protein comprises a sequence selected from:
i) SEQ ID NO:2; and
a sequence which is at least 85 % identical to SEQ ID NO:2.

39. Use of an RBM3 protein as a prognostic marker.

40. Use according to claim 39, wherein said prognostic marker is a prognostic marker for a cancer.

41. Use according to claim 40, wherein said cancer is a breast cancer.

42. Use of an RBM3 protein, or an antigenically active fragment thereof, in the manufacture of a prognostic agent for establishing a prognosis for a mammalian subject having a breast cancer.

43. Use according any one of claims 39-42, wherein the amino acid sequence of the RBM3 protein comprises a sequence selected from:
i) SEQ ID NO:1; and
ii) a sequence which is at least 85 % identical to SEQ ID NO:1.

44. Use according any one of claims 39-42, wherein the amino acid sequence of the RBM3 protein comprises a sequence selected from:
i) SEQ ID NO:2; and
ii) a sequence which is at least 85 % identical to SEQ ID NO:2.

45. Affinity ligand capable of selective interaction with an RBM3 protein.

46. Affinity ligand according to claim 45, which is an antibody or a fragment or a derivative thereof.

47. Affinity ligand according to claim 46, which is obtainable by a process comprising a step of immunizing an animal with a protein whose amino acid sequence comprises the sequence SEQ ID NO:1.

48. Affinity ligand according to any one of claims 45-47 for establishing a prognosis for a mammalian subject having a breast cancer.

49. Use of an affinity ligand according to any one of claims 45-47 as a prognostic agent.

50. Use of an affinity ligand according to any one of claims 45-47 for establishing a prognosis for a mammalian subject having a breast cancer.

51. Use of an affinity ligand according to any one of claims 45-47 in the manufacture of a prognostic agent for establishing a prognosis for a mammalian subject having a breast cancer.

52. Endocrine treatment product for use in the treatment of a mammalian subject having a breast cancer, wherein said subject is diagnosed as hormone receptor positive and RBM3 protein low.

53. Use of an endocrine treatment product in the manufacture of a medicament for treatment of a mammalian subject having a breast cancer, wherein said subject is diagnosed as hormone receptor positive and RBM3 protein low.


SEQUENCE LISTING

<110>  Atlas Antibodies AB

<120>  Breast cancer prognostics

<130>  21035530

<160>  3

<170>  PatentIn version 3.3

<210>  1
<211>  134
<212>  PRT
<213>  Homo sapiens

<400>  1

Asp Glu Gln Ala Leu Glu Asp His Phe Ser Ser Phe Gly Pro Ile Ser
1               5                   10                  15

Glu Val Val Val Val Lys Asp Arg Glu Thr Gln Arg Ser Arg Gly Phe
            20                  25                  30

Gly Phe Ile Thr Phe Thr Asn Pro Glu His Ala Ser Val Ala Met Arg
        35                  40                  45

Ala Met Asn Gly Glu Ser Leu Asp Gly Arg Gln Ile Arg Val Asp His
    50                  55                  60

Ala Gly Lys Ser Ala Arg Gly Thr Arg Gly Gly Gly Phe Gly Ala His
65                  70                  75                  80

Gly Arg Gly Arg Ser Tyr Ser Arg Gly Gly Gly Asp Gln Gly Tyr Gly
                85                  90                  95

Ser Gly Arg Tyr Tyr Asp Ser Arg Pro Gly Gly Tyr Gly Tyr Gly Tyr
            100                 105                 110

Gly Arg Ser Arg Asp Tyr Asn Gly Arg Asn Gln Gly Gly Tyr Asp Arg
        115                 120                 125

Tyr Ser Gly Gly Asn Tyr
    130

<210>  2
<211>  157
<212>  PRT
<213>  Homo sapiens

<400>  2

Met Ser Ser Glu Glu Gly Lys Leu Phe Val Gly Gly Leu Asn Phe Asn
1               5                   10                  15

Thr Asp Glu Gln Ala Leu Glu Asp His Phe Ser Ser Phe Gly Pro Ile
            20                  25                  30

Ser Glu Val Val Val Val Lys Asp Arg Glu Thr Gln Arg Ser Arg Gly
        35                  40                  45

Phe Gly Phe Ile Thr Phe Thr Asn Pro Glu His Ala Ser Val Ala Met
    50                  55                  60

Arg Ala Met Asn Gly Glu Ser Leu Asp Gly Arg Gln Ile Arg Val Asp
65                  70                  75                  80

His Ala Gly Lys Ser Ala Arg Gly Thr Arg Gly Gly Gly Phe Gly Ala
                85                  90                  95

His Gly Arg Gly Arg Ser Tyr Ser Arg Gly Gly Gly Asp Gln Gly Tyr
            100                 105                 110

Gly Ser Gly Arg Tyr Tyr Asp Ser Arg Pro Gly Gly Tyr Gly Tyr Gly
        115                 120                 125

Tyr Gly Arg Ser Arg Asp Tyr Asn Gly Arg Asn Gln Gly Gly Tyr Asp
    130                 135                 140

Arg Tyr Ser Gly Gly Asn Tyr Arg Asp Asn Tyr Asp Asn
145                 150                 155

<210>  3
<211>  1130
<212>  DNA
<213>  Homo sapiens

<400>  3
atgcgcaatg tggcccccta atggtggctg cgctgagcca gctcctcaga ttaccacctt      60

attggccgcc tttctcagct tttctgtagt tacccatatt ttgttcctct ttcttgtcta     120

ttttctgtgc tttttctctg ctttccgtct cgctattttc tcacatctcc attttctttc     180

tccttcctgc caccattctt catgttcttc ccacaggact tgaactgcca tgtcctctga     240

agaaggaaag ctcttcgtgg gagggctcaa ctttaacacc gacgagcagg cactggaaga     300

ccacttcagc agtttcggac ctatctctga ggtggtcgtt gtcaaggacc gggagactca     360

gcggtccagg ggttttggtt tcatcacctt caccaaccca gagcatgctt cagttgccat     420

gagagccatg aacggagagt ctctggatgg tcgtcagatc cgtgtggatc atgcaggcaa     480

gtctgctcgg ggaaccagag gaggtggctt tggggcccat gggcgtggtc gcagctactc     540

tagaggtggt ggggaccagg gctatgggag tggcaggtat tatgacagtc gacctggagg     600

gtatggatat ggatatggac gttccagaga ctataatggc agaaaccagg gtggttatga     660

ccgctactca ggaggaaatt acagagacaa ttatgacaac tgaaatgaga catgcacata     720

atatagatac acaaggaata atttctgatc caggatcgtc cttccaaatg gctgtattta     780

taaaggtttt tggagctgca ccgaagcatc ttattttata gtatatcaac cttttgtttt     840

taaattgacc tgccaaggta gctgaagacc ttttagacag ttccatcttt ttttttaaat     900

tttttctgcc tatttaaaga caaattatgg gacgtttgta gaacctgagt atttttcttt 960
ttaccagttt tttagtttga gctcttaggt ttattggagc tagcaataat tggttctggc 1020
aagtttggcc agactgactt caaaaaatta atgtgtatcc agggacattt taaaaacctg 1080
tacacagtgt ttattgtggt taggaagcaa tttcccaatg tacctataag 1130